# EUROPÄISCHE PATENTANMELDUNG

(11) **EP 0 555 824 A1**
(43) Veröffentlichungstag der Anmeldung: **18.08.1993**
(21) Anmeldenummer: 93102052.3
(22) Anmeldetag: 10.02.1993
(51) Int. Cl.: C07D 235/08, C07D 403/12, C07D 405/12, C07D 401/06, C07D 417/14, C07D 417/06, C07D 401/12, C07D 413/06, A61K 31/415, A61K 31/55, A61K 31/535

(54) **N-alpha-arylsulfonylierte Benzimidazolyl-alaninamid Derivate, diese enthaltende Arzneimittel sowie Verfahren zu ihrer Herstellung**

(30) Priorität: 13.02.1992 DE 4204270
(71) Anmelder: Dr. Karl Thomae GmbH, D-88397 Biberach (DE)
(72) Erfinder: Heckel, Armin, Dr. Dipl.-Chem., W-7950 Biberach 1 (DE); Sauter, Robert, Dr. Dipl.-Chem., W-7958 Laupheim (DE); Psiorz, Manfred, Dr. Dipl.-Chem., W-6507 Ingelheim/Rhein (DE); Binder, Klaus, Dr. Dipl.-Biol., W-7951 Warthausen (DE); Müller, Thomas, Dr. Dipl.-Chem., W-7950 Biberach (DE); Zimmermann, Rainer, Dipl.-Biochem., W-7951 Mittelbiberach (DE)

(57) **Zusammenfassung**

Die Erfindung betrifft Benzimidazolylderivate der allgemeinen Formel 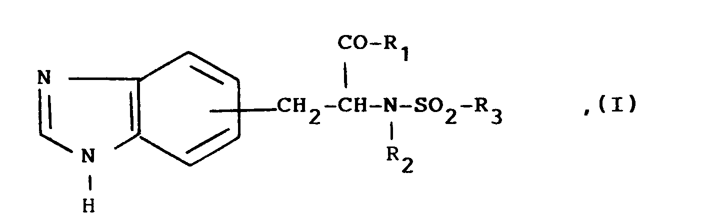
in der
R₁ eine durch Alkylgruppen disubstituierte Aminogruppe, in welcher eine Alkylgruppe durch eine Phenylgruppe substituiert sein kann,
eine gegebenenfalls durch eine Phenyl-, Hydroxy-, Carboxy-, Alkylcarbonyl-, Aminocarbonyl-, Cyano- oder N-Alkanoyl-alkyl-aminogruppe substituierte Pyrrolidino-, Piperidino- oder Hexamethyleniminogruppe, wobei die Hydroxygruppe nicht in α-Position zu dem Ringstickstoffatom stehen kann, eine der vorstehend erwähnten Piperidinogruppen zusätzlich durch eine Alkylgruppe substituiert und zusätzlich die Methylengruppe in 4-Stellung der Piperidinogruppe durch ein Sauerstoffatom, eine Carbonyl-, Sulfinyl-, Imino- oder N-Alkyl-iminogruppe oder eine Ethylengruppe in 3-, 4-Stellung der Piperidinogruppe oder in 4-, 5-Stellung der Hexamethyleniminogruppe durch eine Ethenylen-, Thiophenylen- oder Thiazolylengruppe ersetzt sein kann,
eine durch zwei oder drei Alkylgruppen substituierte Piperidinogruppe, in welcher die Alkylsubstituenten gleich oder verschieden sein können,
eine gegebenenfalls in 2-Stellung durch eine Aminogruppe substituierte Tetrahydro-4H-thiazolo[4,5-d]azepin-6-yl- oder Tetrahydro-thiazolo[4',5':5,4]thieno[3,2-c]pyridin-7-yl-gruppe,
R₂ ein Wasserstoffatom oder eine Alkylgruppe und
R₃ eine gegebenenfalls durch ein Fluor-, Chlor- oder Bromatom, durch eine Alkyl-, Nitro-, Amino-, Alkylamino-, Dialkylamino-, Phenyl- oder Cyclohexylgruppe substituierte Phenylgruppe, wobei der Phenylsubstituent ebenfalls durch ein Fluor-, Chlor- oder Bromatom, durch eine Nitro- oder Aminogruppe substituiert sein kann,
eine durch Fluor-, Chlor- oder Bromatome, durch Alkyl- oder Alkoxygruppen disubstituierte Phenylgruppe, wobei einer der Substituenten auch eine Nitro- oder Aminogruppe darstellen kann,
eine durch eine Hydroxy-, Amino-, Alkylamino-, Dialkylamino- oder Pyrrolylgruppe substituierte Phenylgruppe, wobei gleichzeitig die Phenylgruppe durch zwei Chlor- oder Bromatome oder durch zwei Alkylgruppen mit 1 bis 4 Kohlenstoffatomen substituiert ist und die Pyrrolylgruppe durch eine oder zwei Alkylgruppen substituiert sein kann,
eine gegebenenfalls durch Hydroxy-, Alkoxy- oder Dialkylaminogruppen mono- oder disubstituierte Naphthylgruppe,
eine gegebenenfalls durch eine Alkylgruppe substituierte Indanyl-, Chinolyl-, 1,2,3,4-Tetrahydro-chinolyl-, Isochinolyl-, 1,2,3,4-Tetrahydro-isochinolyl-, Carbazol-, 1,2,3,4-Tetrahydrocarbazol- oder Dibenzofuranylgruppe, wobei eine Iminogruppe zusätzlich durch eine Alkylgruppe, welche gleichzeitig durch eine Carboxy- oder Alkoxycarbonylgruppe substituiert sein kann, substituiert sein kann,
wobei, soweit nichts anderes erwähnt wurde, die bei der Definition der Reste R₁ bis R₃ erwähnten Alkyl-, Alkanoyl- und Alkoxyteile jeweils 1 bis 3 Kohlenstoffatome enthalten können,
deren Gemische von Stellungsisomeren sowie deren Salze, welche wertvolle pharmakologische Eigenschaften aufweisen, insbesondere eine die Thrombinzeit verlängernde Wirkung, eine thrombinhemmende Wirkung und eine Hemmwirkung auf verwandte Serinproteasen wie Trypsin, diese Verbindung enthaltende Arzneimittel und Verfahren zu ihrer Herstellung.

## Beschreibung

Gegenstand der vorliegenden Erfindung sind Benzimidazolyl-derivate der allgemeinen Formel 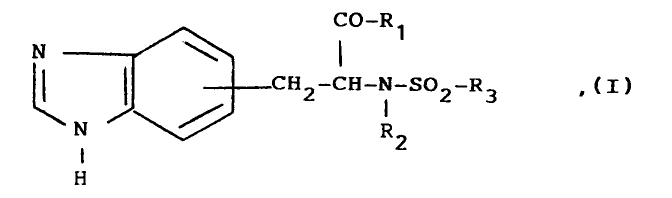
deren Gemische von Stellungsisomeren sowie deren Salze, insbesondere deren physiologisch verträglichen Salzen mit anorganischen oder organischen Säuren oder Basen, welche wertvolle pharmakologische Eigenschaften aufweisen, insbesondere eine die Thrombinzeit verlängernde Wirkung, eine thrombinhemmende Wirkung und eine Hemmwirkung auf verwandte Serinproteasen wie Trypsin, diese Verbindung enthaltende Arzneimittel und Verfahren zu ihrer Herstellung.

In der obigen allgemeinen Formel I bedeutet
R₁ eine durch Alkylgruppen disubstituierte Aminogruppe, in welcher eine Alkylgruppe durch eine Phenylgruppe substituiert sein kann,
eine gegebenenfalls durch eine Phenyl-, Hydroxy-, Carboxy-, Alkylcarbonyl-, Aminocarbonyl-, Cyano- oder N-Alkanoyl-alkylaminogruppe substituierte Pyrrolidino-, Piperidino- oder Hexamethyleniminogruppe, wobei die Hydroxygruppe nicht in α-Position zu dem Ringstickstoffatom stehen kann, eine der vorstehend erwähnten Piperidinogruppen zusätzlich durch eine Alkylgruppe substituiert und zusätzlich die Methylengruppe in 4-Stellung der Piperidinogruppe durch ein Sauerstoffatom, eine Carbonyl-, Sulfinyl-, Imino- oder N-Alkyl-iminogruppe oder eine Ethylengruppe in 3-, 4-Stellung der Piperidinogruppe oder in 4-, 5-Stellung der Hexamethyleniminogruppe durch eine Ethenylen-, Thiophenylen- oder Thiazolylengruppe ersetzt sein kann,
eine durch zwei oder drei Alkylgruppen substituierte Piperidinogruppe, in welcher die Alkylsubstituenten gleich oder verschieden sein können,
eine gegebenenfalls in 2-Stellung durch eine Aminogruppe substituierte Tetrahydro-4H-thiazolo[4,5-d]azepin-6-yl- oder Tetrahydro-thiazolo[4',5':5,4]thieno[3,2-c]pyridin-7-yl-gruppe,
R₂ ein Wasserstoffatom oder eine Alkylgruppe und
R₃ eine gegebenenfalls durch ein Fluor-, Chlor- oder Bromatom, durch eine Alkyl-, Nitro-, Amino-, Alkylamino-, Dialkylamino-, Phenyl- oder Cyclohexylgruppe substituierte Phenylgruppe, wobei der Phenylsubstituent ebenfalls durch ein Fluor-, Chlor- oder Bromatom, durch eine Nitro- oder Aminogruppe substituiert sein kann,
eine durch Fluor-, Chlor- oder Bromatome, durch Alkyl- oder Alkoxygruppen disubstituierte Phenylgruppe, wobei einer der Substituenten auch eine Nitro- oder Aminogruppe darstellen kann,
eine durch eine Hydroxy-, Amino-, Alkylamino-, Dialkylamino- oder Pyrrolylgruppe substituierte Phenylgruppe, wobei gleichzeitig die Phenylgruppe durch zwei Chlor- oder Bromatome oder durch zwei Alkylgruppen mit 1 bis 4 Kohlenstoffatomen substituiert ist und die Pyrrolylgruppe durch eine oder zwei Alkylgruppen substituiert sein kann,
eine gegebenenfalls durch Hydroxy-, Alkoxy- oder Dialkylaminogruppen mono- oder disubstituierte Naphthylgruppe,
eine gegebenenfalls durch eine Alkylgruppe substituierte Indanyl-, Chinolyl-, 1,2,3,4-Tetrahydro-chinolyl-, Isochinolyl-, 1,2,3,4-Tetrahydro-isochinolyl-, Carbazol-, 1,2,3,4-Tetrahydrocarbazol- oder Dibenzofuranylgruppe, wobei eine Iminogruppe zusätzlich durch eine Alkylgruppe, welche gleichzeitig durch eine Carboxy- oder Alkoxycarbonylgruppe substituiert sein kann, substituiert sein kann,
wobei, soweit nichts anderes erwähnt wurde, die bei der Definition der Reste R₁ bis R₃ erwähnten Alkyl-, Alkanoyl- und Alkoxyteile jeweils 1 bis 3 Kohlenstoffatome enthalten können.

Für die bei der Definition der Reste R₁ bis R₃ erwähnten Bedeutungen kommt beispielsweise
für R₁ die Bedeutung der Dimethylamino-, Diethylamino-, Di-n-propylamino-, Diisopropylamino-, N-Ethyl-methylamino-, N-Benzyl-methylamino-, N-Benzyl-ethylamino-, N-Benzyl-isopropylamino-, Pyrrolidino-, 3-Methyl-pyrrolidino-, 3-Ethyl-pyrrolidino-, 3-Isopropyl-pyrrolidino-, Piperidino-, 4-Methyl-piperidino-, 4-Ethyl-piperidino-, 4-n-Propyl-piperidino-, 4,4-Dimethyl-piperidino-, 4,4-Diethyl-piperidino-, 2,4,6-Trimethyl-piperidino-, N-Methyl-indan-1-yl-amino-, N-Ethyl-indan-1-yl-amino-, N-Isopropyl-indan-1-yl-amino-, 5,6,7,8-Tetrahydro-thiazolo(4',5':5,4]thieno[3,2-c]pyridino-, 4,5,6,7-Tetrahydro-thieno[3,2-c]pyridino-, 5,6,7,8-Tetrahydro-4H-thiazolo[4,5-d]azepino-, 5,6,7,8-Tetrahydro-4H-thieno[2,3-d]azepino-, 5,6,7,8-Tetrahydro-thiazolo[4',5':5,4]thieno[3,2-c]pyridino-, 2-Amino-5,6,7,8-tetrahydro-thiazolo[4',5':5,4]thieno[3,2-c]pyridino-, Morpholino-, 2-Methyl-morpholino-, 2-Ethyl-morpholino-, 2-Phenyl-morpholino-, Thiomorpholino-, 1-Oxido-thiomorpholino-, 4-(N-Acetyl-methylamino)piperidino-, 4-(N-Acetyl-ethylamino)-piperidino-, 4-(N-Acetyl-n-propylamino)-piperidino-, 4-(N-Propionyl-methylamino)-piperidino-, 4-(N-Propionyl-ethylamino)-piperidino-, 4-(N-Propionyl-n-propylamino)-piperidino-, 2,3,4,5,6,7-Hexahydro-1H-azepino-, 1,2,3,6-Tetrahydro-pyridino-, 4-Methyl-1,2,3,6-tetrahydro-pyridino-, 4-Ethyl-1,2,3,6-tetrahydro-pyridino-, 4-Isopropyl-1,2,3,6-tetrahydro-pyridino-, 4-Phenyl-1,2,3,6-tetrahydro-pyridino-, 2-Carboxy-piperidino-, 2-Carbmethoxy-piperidino-, 2-Carbethoxy-piperidino-, 2-Carbisopropoxy-piperidino-, 2-Carboxy-4-methyl-piperidino-, 2-Carboxy-4-ethyl-piperidino-, 2-Carboxy-4-n-propyl-piperidino-, 2-Carbmethoxy-4-methyl-piperidino-, 2-Carbmethoxy-4-ethyl-piperidino-, 2-Carbmethoxy-4-n-propyl-piperidino-, 2-Carbethoxy-4-methyl-piperidino-, 2-Carbethoxy-4-ethyl-piperidino-, 2-Carbisopropoxy-4-n-propyl-piperidino-, 2-Carbisopropoxy-4-methyl-piperidino-, 2-Carbisopropoxy-4-ethyl-piperidino-, 2-Carbisopropoxy-4-n-propyl-piperidino-, 4-Amino-piperidino-, 4-Methylamino-piperidino-, 4-Ethylamino-piperidino-, 4-n-Propylamino-piperidino-, 4-Dimethylamino-piperidino-, 4-Diethylamino-piperidino-, 4-Cyano-piperidino-, 4-Hydroxy-piperidino-, 4-Oxo-piperidino-, Piperazino-, 4-Methyl-piperazino-, 4-Ethyl-piperazino-, 4-n-Propyl-piperazino- oder 4-Isopropyl-piperazino-gruppe,
für R₂ die des Wasserstoffatoms, der Methyl-, Ethyl-, n-Propyl- oder Isopropylgruppe und
für R₃ die der Phenyl-, 2-Fluorphenyl-, 3-Fluorphenyl-, 4-Fluorphenyl-, 2-Chlorphenyl-, 3-Chlorphenyl-, 4-Chlorphenyl-, 2-Bromphenyl-, 3-Bromphenyl-, 4-Bromphenyl-, 2-Methylphenyl-, 3-Methylphenyl-, 4-Methylphenyl-, 2-Ethylphenyl-, 3-Ethylphenyl-, 4-Ethylphenyl-, 2-Isopropylphenyl-, 3-Isopropylphenyl-, 4-Isopropylphenyl-, 2-Nitrophenyl-, 3-Nitrophenyl-, 4-Nitrophenyl-, 2-Aminophenyl-, 3-Aminophenyl-, 4-Aminophenyl-, 2-Methylaminophenyl-, 3-Methylaminophenyl-, 4-Methylaminophenyl-, 2-Dimethylaminophenyl-, 3-Dimethylaminophenyl-, 4-Dimethylaminophenyl-, 2-Ethylaminophenyl-, 3-Ethylaminophenyl-, 4-Ethylaminophenyl-, 2-Diethylaminophenyl-, 3-Diethylaminophenyl-, 4-Diethylaminophenyl-, 2-Biphenylyl-, 3-Biphenylyl-, 4-Biphenylyl-, 2-Cyclohexylphenyl-, 3-Cyclohexylphenyl-, 4-Cyclohexylphenyl-, 2'-Fluor-4-biphenylyl-, 3'-Fluor-4-biphenylyl-, 2'-Chlor-4-biphenylyl-, 3'-Chlor-4-biphenylyl-, 2'-Brom-4-biphenylyl-, 3'-Brom-4-biphenylyl-, 2'-Methyl-4-biphenylyl-, 3'-Methyl-4-biphenylyl-, 2'-Nitro-4-biphenylyl-, 3'-Nitro-4-biphenylyl-, 2'-Amino-4-biphenylyl-, 3'-Amino-4-biphenylyl-, 2'-Methylamino-4-biphenylyl-, 3'-Methylamino-4-biphenylyl-, 2'-Dimethylamino-4-biphenylyl-, 3'-Dimethylamino-4-biphenylyl-, 3,4-Difluorphenyl-, 3,4-Dichlorphenyl-, 3,4-Dibromphenyl-, 2,5-Dimethylphenyl-, 3,4-Dimethylphenyl-, 3,4-Dimethoxyphenyl-, 4-Chlor-3-nitro-phenyl-, 4-Brom-3-nitro-phenyl-, 4-Hydroxy-3,5-di-tert.butylphenyl-, 4-Amino-3,5-dichlorphenyl-, 4-Amino-3,5-dibromphenyl-, 4-Methylamino-3,5-dichlorphenyl-, 4-Methylamino-3,5-dibromphenyl-, 4-Ethylamino-3,5-dichlorphenyl-, 4-Ethylamino-3,5-dibromphenyl-, 4-Dimethylamino-3,5-dichlorphenyl-, 4-Dimethylamino-3,5-dibromphenyl-, 4-Diethylamino-3,5-dichlorphenyl-, 4-Diethylamino-3,5-dibromphenyl-, 3,5-Dichlor-4-pyrrolyl-phenyl-, 3,5-Dibrom-4-pyrrolyl-phenyl-, Naphth-1-yl-, Naphth-2-yl-, 1-Hydroxy-naphth-1-yl-, 1-Methoxy-naphth-1-yl-, 8-Hydroxy-naphth-1-yl-, 8-Methoxy-naphth-1-yl-, 6,7-Dimethoxy-naphth-1-yl-, 5-Amino-naphth-1-yl-, 5-Methylamino-naphth-1-yl-, 5-Dimethylamino-naphth-1-yl-, Dibenzofuryl-(2)-, Dibenzofuryl-(3)-, Carbazol-9-yl-, 9-Methyl-carbazol-3-yl-, 9-Ethyl-carbazol-3-yl-, 9-Hydroxycarbonylmethyl-carbazol-3-yl-, 9-Methoxycarbonylmethyl-carbazol-3-yl-, 9-Ethoxycarbonylmethyl-carbazol-3-yl-, 9-n-Propoxycarbonylmethyl-carbazol-3-yl-, 9-Methyl-1,2,3,4-tetrahydro-carbazol-6-yl-, 1,2,3,4-Tetrahydro-chinolin-8-yl-, 1,2,3,4-Tetrahydro-chinolin-5-yl-, 3-Methyl-1,2,3,4-tetrahydro-chinolin-8-yl-, 3-Methyl-1,2,3,4-tetrahydro-chinolin-5-yl-, 3-Ethyl-1,2,3,4-tetrahydro-chinolin-8-yl-, 3-Ethyl-1,2,3,4-tetrahydro-chinolin-5-yl-, 2-Acetyl-1,2,3,4-tetrahydro-chinolin-5-yl-, 2-Propionyl-1,2,3,4-tetrahydro-chinolin-5-yl-, 2-Methoxycarbonyl-1,2,3,4-tetrahydro-chinolin-5-yl-, 2-Ethoxycarbonyl-1,2,3,4-tetrahydro-chinolin-5-yl-, 2-Isopropoxycarbonyl-1,2,3,4-tetrahydro-chinolin-5-yl- oder Chinolin-8-yl-gruppe in Betracht.

Bevorzugte Verbindungen der obigen allgemeinen Formel I sind jedoch diejenigen, in denen
R₁ eine N-Methyl-benzylaminogruppe, eine gegebenenfalls in 4-Stellung durch eine Alkylgruppe mit 1 bis 3 Kohlenstoffatomen, durch eine Hydroxy-, Cyano-, Aminocarbonyl-, Methylamino- oder N-Acetyl-methylaminogruppe substituierte Piperidinogruppe, eine durch zwei oder drei Methylgruppen substituierte Piperidinogruppe, eine durch eine Carboxy-, Methoxycarbonyl- oder Ethoxycarbonylgruppe substituierte 4-Methyl-piperidinogruppe, eine gegebenenfalls in 2-Stellung durch eine Phenylgruppe substituierte Morpholinogruppe, eine 4-Oxo-pyrrolidino-, 1-Oxido-thiomorpholino-, 2,3,4,5,6,7-Hexahydro-1H-azepino-, 4-Methyl-piperazino-, 5,6,7,8-Tetrahydro-thiazolo[4',5':5,4]thieno[3,2-c]pyridino-, 4,5,6,7-Tetrahydro-thieno[3,2-c]pyridino-, 5,6,7,8-Tetrahydro-4H-thiazolo[4,5-d]azepino-, 5,6,7,8-Tetrahydro-4H-thieno[2,3-d]azepino-, 5,6,7,8-Tetrahydro-thiazolo[4',5':5,4]thieno[3,2-c]pyridino- oder 2-Amino-5,6,7,8-tetrahydro-thiazolo[4',5':5,4]thieno[3,2-c]pyr idinogruppe,
R₂ ein Wasserstoffatom oder eine Methylgruppe und
R₃ eine gegebenenfalls durch ein Chloratom, durch eine Methyl-, Nitro-, Amino-, Phenyl- oder Cyclohexylgruppe substituierte Phenylgruppe, wobei der Phenylsubstituent ebenfalls durch ein Fluoratom, durch eine Nitro- oder Aminogruppe substituiert sein kann,
eine durch Chloratome oder durch Methylgruppen disubstituierte Phenylgruppe, wobei die Substituenten gleich oder verschieden sein können und zusätzlich auch einer der Substituenten eine Nitro- oder Aminogruppe darstellen kann,
eine durch eine Hydroxy-, Amino-, Methylamino-, Ethylamino-, Dimethylamino- oder Pyrrolylgruppe substituierte Phenylgruppe, wobei gleichzeitig die Phenylgruppe durch zwei Chloratome oder durch zwei Alkylgruppen mit 1 bis 4 Kohlenstoffatomen substituiert ist,
eine gegebenenfalls durch Hydroxy-, Methoxy- oder Dimethylaminogruppen mono- oder disubstituierte Naphthylgruppe,
eine gegebenenfalls durch eine Methylgruppe substituierte Indanyl-, Chinolyl-, 1,2,3,4-Tetrahydro-chinolyl-, Isochinolyl-, 1,2,3,4-Tetrahydro-isochinolyl-, Dibenzofuryl-, Carbazolyl- oder 1,2,3,4-Tetrahydro-carbazolylgruppe, wobei eine Iminogruppe zusätzlich durch eine Methyl-, Ethyl-, Methoxycarbonyl-, Ethoxycarbonyl-, Hydroxycarbonylmethyl-, Methoxycarbonylmethyl- oder Ethoxycarbonylmethylgruppe substituiert sein kann, bedeuten,
deren Enantiomere und deren Salze.

Besonders bevorzugte Verbindungen sind die in 5-Stellung substituierten Benzimidazolylderivate der obigen allgemeinen Formel I, in der
R₁ eine gegebenenfalls in 4-Stellung durch eine Alkylgruppe mit 1 bis 3 Kohlenstoffatomen substituierte Piperidinogruppe, eine in 2-Stellung durch eine Carboxy-, Methoxycarbonyl- oder Ethoxycarbonylgruppe substituierte 4-Methyl-piperidinogruppe, eine 4-Oxo-piperidino-, 2,3,4,5,6,7-Hexahydro-1H-azepino-, 4-Methyl-piperazino-, 4-Methyl-1,2,3,6-tetrahydro-pyridino-, 4,5,6,7-Tetrahydro-thieno[3,2-c]pyridino-, 5,6,7,8-Tetrahydro-4H-thiazolo[4,5-d]azepino- oder 5,6,7,8-Tetrahydro-4H-thieno[2,3-d]azepino-gruppe,
R₂ ein Wasserstoffatom und
R₃ eine durch eine Hydroxy-, Amino-, Methylamino-, Ethylamino-, Dimethylamino- oder Pyrrolylgruppe substituierte Phenylgruppe, wobei gleichzeitig die Phenylgruppe durch zwei Chloratome oder durch zwei tert.Butylgruppen substituiert ist,
eine 4-Biphenylylgruppe,
eine durch eine Dimethylaminogruppe substituierte Naphthylgruppe,
eine gegebenenfalls durch eine Methylgruppe substituierte 1,2,3,4-Tetrahydro-chinolyl-, Carbazolyl-, 1,2,3,4-Tetrahydrocarbazol- oder Dibenzofurylgruppe, wobei eine Iminogruppe zusätzlich durch eine Methyl-, Methoxycarbonyl-, Ethoxycarbonyl-, Hydroxycarbonylmethyl-, Methoxycarbonylmethyl- oder Ethoxycarbonylmethylgruppe substituiert sein kann, bedeuten,
deren 1-, 3-Isomerengemische und deren Enantiomere sowie deren Salze.

Erfindungsgemäß erhält man die neuen Verbindungen nach folgenden Verfahren:
a) Cyclisierung einer Verbindung der allgemeinen Formel 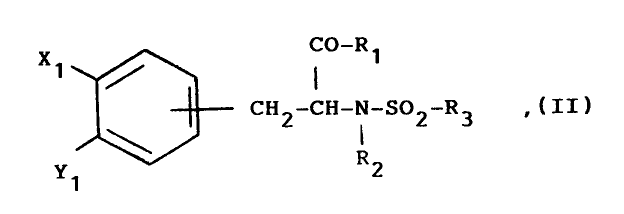 in der
   R₁, R₂ und R₃ wie eingangs definiert sind,
   einer der Reste X₁ oder Y₁ eine Formylaminogruppe und
   der andere der Reste X₁ oder Y₁ eine Aminogruppe darstellen.
   Die Cyclisierung wird zweckmäßigerweise in einem Lösungsmittel oder Lösungsmittelgemisch wie Ethanol, Isopropanol, Benzol, Chlorbenzol, Toluol, Xylol, Glycol, Glycolmonomethylether, Diethylenglycoldimethylether, Sulfolan, Dimethylformamid, Tetralin oder in Ameisensäure bei Temperaturen zwischen 0 und 100°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/oder Kondensationsmittel durchgeführt werden.
   Besonders vorteilhaft wird die Umsetzung jedoch in der Weise durchgeführt, daß eine Verbindung der Formel II im Reaktionsgemisch durch Reduktion einer entsprechenden o-Nitro-aminoverbindung gegebenenfalls in Gegenwart von Ameisensäure oder durch Acylierung einer entsprechenden o-Diaminoverbindung hergestellt wird.
b) Umsetzung einer Verbindung der allgemeinen Formel 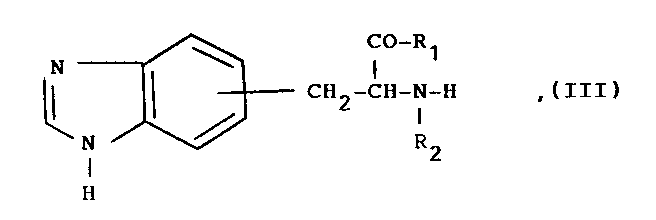 in der
   R₁ und R₂ wie eingangs definiert sind, mit einer Verbindung der allgemeinen Formel

   Z₁-SO₂-R₃ , (IV)

   in der
   R₃ wie eingangs definiert ist und
   Z₁ eine nukleophile Austrittsgruppe wie ein Halogenatom, eine Alkoxy-, Alkylthio- oder Benzyloxygruppe, z. B. ein Chlor- oder Bromatom, eine Methoxy-, Ethoxy-, Methylthio-, Ethylthio- oder Benzyloxygruppe, darstellt.
   Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.
c) Umsetzung einer Verbindung der allgemeinen Formel 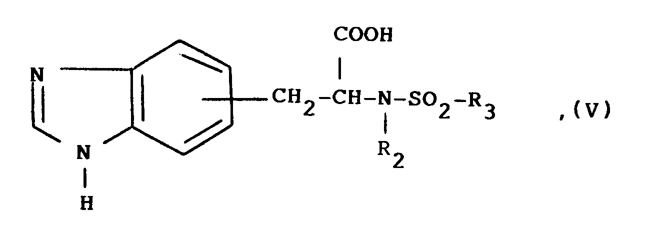 in der
   R₂ und R₃ wie eingangs definiert sind, oder dessen reaktionsfähiges Derivat mit einer Verbindung der allgemeinen Formel

   H-R₁ , (VI)

   in der
   R₁ wie eingangs definiert ist.
   Die Umsetzung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, besonders vorteilhaft jedoch in einem Überschuß der eingesetzten Verbindung der allgemeinen Formel VI, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxy-succinimid, N,N'-Carbonyldiimidazol oder N,N'-Thionyldiimidazol oder Triphenylphosphin/Tetrachlorkohlenstoff, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25°C und 250°C, vorzugsweise jedoch bei Temperaturen zwischen -10°C und der Siedetemperatur des verwendeten Lösungsmittels, durchgeführt.
d) Zur Herstellung von Verbindungen der allgemeinen Formel I, in der R₃ eine Alkylamino- oder Dialkylaminogruppe enthält:
   Reduktive Aminierung einer Verbindung der allgemeinen Formel 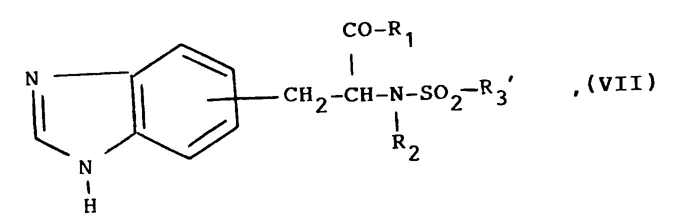 in der
   R₁ und R₂ wie eingangs definiert sind und
   R₃' eine durch eine Amino- oder Alkylaminogruppe substituierte Phenylgruppe, die zusätzlich durch zwei Chlor- oder Bromatome oder durch zwei Alkylgruppen mit 1 bis 4 Kohlenstoffatomen substituiert sein kann, darstellt, mit einem Alkanal mit 1 bis 3 Kohlenstoffatomen.

Die reduktive Aminierung wird in einem geeigneten Lösungsmittel wie Methanol, Ethanol, Totrahydrofuran, Dioxan oder Acetonitril in Gegenwart eines geeigneten Reduktionsmittels wie Ameisensäure oder einem geeigneten komplexen Metallhydrid, vorzugsweise jedoch in Gegenwart von Natriumcyanborhydrid bei einem pH-Wert von 5 bis 7, bei Temperaturen zwischen 0 und 50°C, vorzugsweise jedoch bei Raumtemperatur, durchgeführt.

Erhält man erfindungsgemäß eine Verbindung der allgemeinen Formel I, in der R₃ eine Aminogruppe enthält, so kann diese mittels Umsetzung eines entsprechenden Furans in eine entsprechende Verbindung der allgemeinen Formel I, in der R₃ einen entsprechenden Pyrrolylrest enthält, übergeführt werden oder
eine Verbindung der allgemeinen Formel I, in der R₃ eine Nitrogruppe enthält, so kann diese mittels Reduktion in eine entsprechende Verbindung der allgemeinen Formel I, in der R₃ eine Aminogruppe enthält, übergeführt werden oder
eine Verbindung der allgemeinen Formel I, die eine veresterte Carboxygruppe enthält, so kann diese mittels Hydrolyse in eine entsprechende Carboxyverbindung übergeführt werden oder
eine Verbindung der allgemeinen Formel I, in der R₁ eine durch eine Aminocarbonylgruppe substituierte Pyrrolidino-, Piperidino- oder Hexamethyleniminogruppe darstellt, so kann diese mittels Dehydratisierung in eine entsprechende Cyanoverbindung übergeführt werden oder
eine Verbindung der allgemeinen Formel I, in der R₁ oder R₂ oder R₁ und R₂ eine Carbonylgruppe enthält, so kann diese mittels Reduktion in eine entsprechende Hydroxymethylenverbindung übergeführt werden oder
eine Verbindung der allgemeinen Formel I, in der R₃ einen ankondensierten Pyridinring enthält, so kann diese mittels katalytischer Hydrierung in eine entsprechende Tetrahydro-Verbindung übergeführt werden.

Die nachträgliche Amidierung wird zweckmäßigerweise in einem Lösungsmittel wie Methanol/Eisessig, Ethanol/Eisessig oder Dioxan/Propionsäure bei erhöhten Temperaturen, z. B. bei Temperaturen zwischen 50 und 100°C, vorzugsweise jedoch bei der Siedetemperatur des Reaktionsgemisches, durchgeführt.

Die nachträgliche Reduktion der Nitrogruppe wird vorzugsweise in einem Lösungsmittel wie Wasser, Wasser/Ethanol, Methanol, Eisessig, Essigsäurethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit, oder mit Hydrazin in Gegenwart von Raney-Nickel bei Temperaturen zwischen 0 und 80°C, vorzugsweise jedoch bei Temperaturen zwischen 20 und 40°C, durchgeführt.

Die nachträgliche Hydrolye erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches.

Die nachträgliche Dehydratisierung wird vorzugsweise in einem Lösungsmittel wie Benzol, Toluol oder Dichlorbenzol in Gegenwart eines wasserentziehenden Mittels wie Phosphoroxychlorid, Thionylchlorid oder Phosphorpentoxid bei Temperaturen zwischen 25 und 75°C durchgeführt.

Die nachträgliche Reduktion der Carboxygruppe wird in einem geeigneten Lösungsmittel wie Methanol, Äthanol, Äther, Tetrahydrofuran, Dioxan oder Eisessig in Gegenwart von katalytisch angeregtem Wasserstoff, z.B. von Wasserstoff in Gegenwart von Platin oder Palladium/Kohle, und gegebenenfalls in Gegenwart einer Säure wie Salzsäure oder Perchlorsäure oder in Gegenwart eines Metallhydrids wie Natriumborhydrid, Lithium-borhydrid oder Lithiumaluminiumhydrid bei Temperaturen zwischen 0 und 100°C, vorzugsweise bei Temperaturen zwischen 20 und 80°C, durchgeführt.

Die nachträgliche katalytische Hydrierung wird vorzugsweise in einem Lösungsmittel wie Wasser, Wasser/Ethanol, Methanol, Eisessig, Essigsäurethylester oder Dimethylformamid in Gegenwart eines Hydrierungskatalysators wie Raney-Nickel, Platin oder Palladium/Kohle bei Temperaturen zwischen 0 und 80°C, vorzugsweise jedoch bei Temperaturen zwischen 20 und 40°C, durchgeführt.

Ferner können die erhaltenen Verbindungen der allgemeinen Formel I in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsaure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis VII erhält man nach literaturbekannten Verfahren bzw. sind literaturbekannt.

So erhält man beispielsweise eine Verbindung der allgemeinen Formel II durch Reduktion einer entsprechenden o-Amino-nitro-Verbindung, welche man ihrerseits durch Trifluoracetylierung eines entsprechenden 4-Nitro-phenylalanins, Reduktion der Nitrogruppe unter gleichzeitiger Acetylierung, Nitrierung der so erhaltenen acetylierten Verbindung, anschließende Amidierung der so erhaltenen Carbonsäure, Abspaltung der Trifluoracetylgruppe und Sulfonierung des so erhaltenen o-Amino-nitro-phenylalaninamids erhält.

Eine Verbindung der allgemeinen Formeln III, V und VII erhält man durch Reduktion und Cyclisierung eines entsprechenden vorstehend beschriebenen o-Amino-nitro-phenylalaninamids mit Ameisensäure.

Wie bereits eingangs erwähnt, weisen die neuen Verbindungen der allgemeinen Formel I und deren physiologisch verträglichen Salze wertvolle pharmakologische Eigenschaften auf, insbesondere eine die Thrombinzeit verlängernde Wirkung.

Beispielsweise wurden die Verbindungen
- A =: 4-Amino-N-[1-(1H-benzimidazol-5-yl-methyl)-2-(4-ethyl-piperidin-1-yl)-2-oxo-ethyl]-3,5-dichlor-benzolsulfonamid,
- B =: N-[1-(1H-Benzimidazol-5-yl-methyl)-2-(4-ethyl-piperidin-1-yl)-2-oxo-ethyl]-3,5-dichlor-4-ethylamino-benzolsulfonamid,
- C =: 4-Amino-N-[1-(1H-benzimidazol-5-yl-methyl)-2-(4-methyl-piperazin-1-yl)-2-oxo-ethyl]-3,5-dichlor-benzolsulfonamid,
- D =: N-[1-(1H-Benzimidazol-5-yl-methyl)-2-(4-methyl-piperidin-1-yl)-2-oxo-ethyl]-9-methyl-1,2,3,4-tetrahydro-carbazol-6-sulfonamid,
- E =: 4-Amino-N-[1-(1H-benzimidazol-5-yl-methyl)-2-(4,5,6,7-tetrahydro-thieno[3,2-c]pyridin-5-yl)-2-oxo-ethyl]-3,5-dichlor-benzolsulfonamid,
- F =: 4-Amino-N-[1-(1H-benzimidazol-5-yl-methyl)-2-(piperidin-4-on-1-yl)-2-oxo-ethyl]-3,5-dichlor-benzolsulfonamid,
- G =: N-[1-(1H-Benzimidazol-5-yl-methyl)-2-(4-methyl-piperidin-1-yl)-2-oxo-ethyl]-1,2,3,4-tetrahydro-chinolin-8-sulfonamid,
- H =: 4-Amino-N-[1-(1H-benzimidazol-5-yl-methyl)-2-(2,3,4,5,6,7-hexahydro-1H-azepin-1-yl)-2-oxo-ethyl]-3,5-dichlor-benzolsulfonamid,
- I =: N-[1-(1H-Benzimidazol-5-yl-methyl)-2-(4-methyl-piperidin-1-yl)-2-oxo-ethyl]-9-ethyl-carbazol-3-sulfonamid und
- J =: 4-Amino-N-[1-(1H-benzimidazol-5-yl-methyl)-2-(4-methyl-piperidin-1-yl)-2-oxo-ethyl]-3,5-dichlor-benzolsulfonamid
auf ihre Wirkung auf die Thrombinzeit wie folgt untersucht:
- Material:: Plasma, aus humanem Citratblut.
Test-Thrombin (Rind), 30U/ml, Behring Werke, Marburg
Diethylbarbituratacetat-Puffer, ORWH 60/61, Behring Werke, Marburg
Biomatic B10 Koagulometer, Sarstedt

### Durchführung:

Die Bestimmung der Thrombinzeit erfolgte mit einem Biomatic B 10 Koagulometer der Firma Sarstedt.

Als Testsubstanz wurden in die vom Hersteller vorgeschriebenen Teststreifen 0,1 ml humanes Citratplasma und 0,1 ml Diethylbarbiturat Puffer (DBA-Puffer) gegeben. Der Ansatz wurde für eine Minute bei 37°C inkubiert. Durch Zugabe von 0,3 U Test-Thrombin in 0,1 ml DBA-Puffer wurde die Gerinnungsreaktion gestartet. Gerätebedingt erfolgt mit der Eingabe von Thrombin die Messung der Zeit bis zur Gerinnung des Ansatzes. Als Kontrolle dienten Ansätze bei denen 0,1 ml DBA-Puffer zugegeben wurden. Gemäß der Definition wurde über eine Dosis-Wirkungskurve die Substanzkonzentration als effektive Konzentration ermittelt, bei der die Thrombinzeit gegenüber der Kontrolle verdoppelt wurde.

Die nachfolgende Tabelle enthält die gefundenen Werte:

| Substanz | Thrombinzeit (ED₂₀₀ in µM) |
|---|---|
| A | 2,5 |
| B | 1,7 |
| C | 4,8 |
| D | 2,2 |
| E | 2,7 |
| F | 1,9 |
| G | 9,2 |
| H | 4,6 |
| I | 8,3 |
| J | 4,2 |

Desweiteren konnten bei der Applikation der vorstehenden Verbindungen bis zu einer Dosis von 30 mg/kg i.v. oder 300 mg/kg p.o. keine toxischen Nebenwirkungen beobachtet werden. Die Verbindungen sind demnach gut verträglich.

Aufgrund ihrer pharmakologischen Eigenschaften eignen sich die neuen Verbindungen und deren physiologisch verträglichen Salze zur Vorbeugung und Behandlung venöser und arterieller thrombotischer Erkrankungen, wie zum Beispiel der tiefen Beinvenenthrombose, die Verhinderung von Reocclusionen nach Bypass-Operationen oder Angioplastie (PT(C)A = perkutane transluminale koronare Angioplastie), sowie die Occlusion bei peripheren arteriellen Erkrankungen wie Lungenembolie, der disseminierten intravaskulären Gerinnung etc.. Zusätzlich sind die erfindungsgemäßen Verbindungen zur antithrombotischen Unterstützung bei thrombolitischer Behandlung, wie zum Beispiel mit rt-PA oder Streptokinase, geeignet.

Die zur Erzielung einer entsprechenden Wirkung erforderliche Dosierung beträgt zweckmäßigerweise bei intravenöser Gabe 1 bis 50 mg/kg, vorzugsweise 5 bis 30 mg/kg und bei oraler Gabe 5 bis 100 mg/kg, vorzugsweise 10 bis 50 mg/kg, jeweils 1 bis 3 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsprodukte:

### Beispiel I

### N-Trifluoracetyl-4-nitro-phenylalanin

Eine Lösung von 68 g (0,31 Mol) 4-Nitro-phenylalanin-hydrat in 205 ml Trifluoressigsäure wird langsam zu 87,5 ml (0,62 Mol) Trifluoressigsäureanhydrid getropft. Nach einer Stunde Rühren bei 30°C wird in 150 ml Eiswasser gegossen und nach einer weiteren Stunde der ausgefallene Niederschlag abgesaugt.
Ausbeute: 77,5 g (79 % der Theorie),
Schmelzpunkt: 140-141°C

| C₁₁H₉F₃N₂O₅ (306,20) | | | |
|---|---|---|---|
| Ber. x 0,5 H₂O: | C 41,92 | H 3,20 | N 8,89 |
| Gef.: | 42,15 | 3,20 | 8,86 |

### Beispiel II

### N-Trifluoracetyl-4-acetylamino-phenylalanin

Eine Mischung von 60,8 g (0,2 Mol) N-Trifluoracetyl-4-nitrophenylalanin, 250 ml Eisessig, 94 ml (1,0 Mol) Essigsäureanhydrid und 10 g 10%ige Palladium/Kohle wird 5 Stunden bei 50°C und 5 bar Wasserstoff hydriert. Anschließend wird abgesaugt und das Filtrat zur Trockene eingeengt.
Ausbeute: 63,2 g (100 % der Theorie).

### Beispiel III

### N-Trifluoracetyl-4-acetylamino-3-nitro-phenylalanin

63,2 g (0,2 Mol) N-Trifluoracetyl-4-acetylamino-phenylalanin werden in 300 ml Eisessig und 100 ml Acetanhydrid suspendiert. Nach Abkühlen der Lösung auf 10°C werden 1,5 g (0,022 Mol) Natriumnitrit zugegeben und anschließend 33,14 ml (0,8 Mol) rauchende Salpetersäure langsam zugetropft, wobei Lösung eintritt. Nach 2-stündigem Rühren bei 0°C wird auf 1 kg Eis gegeben und 3 mal mit 250 ml Essigester extrahiert. Nach Trocknen der organischen Phase wird eingeengt, der erhaltene Rückstand 2 mal mit einem Gemisch aus 150 ml Essigester und 150 ml Toluol versetzt und wieder eingeengt. Der dann erhaltene Rückstand wird mit 100 ml auf -10°C abgekühltem Essigester versetzt, abgesaugt und mit -10°C abgekühltem Essigester nachgewaschen.
Ausbeute: 50 g (69 % der Theorie),
Schmelzpunkt: 180-185°C

### Beispiel IV

### N-Trifluoracetyl-4-acetylamino-3-nitro-phenylalanyl-(4-methyl-piperidin)

Eine Lösung von 5,3 g (14,6 mMol) N-Trifluoracetyl-4-acetylamino-3-nitro-phenylalanin in 20 ml absolutem Dimethylformamid wird bei 20°C unter Rühren mit 2,4 g (14,6 mMol) Carbonyldiimidazol versetzt. Nach 1-stündigem Rühren bei 20°C werden 8,4 ml (0,069 Mol) 4-Methyl-piperidin zugegeben und 2 Stunden bei 20°C gerührt. Anschließend wird zur Trockene eingeengt, der Rückstand in 500 ml Essigester aufgenommen und 3 x mit 2N Salzsäure extrahiert. Nach Waschen der organischen Phase mit gesättigter Kochsalzlösung wird getrocknet und erneut eingeengt. Der erhaltene Rückstand wird in Ether digeriert und abgesaugt.
Ausbeute: 5,0 g (77,0 % der Theorie),
Schmelzpunkt: 174-178°C

| C₁₉H₂₃F₃N₄O₅ (444,40) | | | |
|---|---|---|---|
| Ber.: | C 51,35 | H 5,22 | N 12,61 |
| Gef.: | 51,09 | 5,10 | 12,40 |

### Beispiel V

### 4-Amino-3-nitro-phenylalanyl-(4-methyl-piperidin)

Zu einer Suspension von 4,85 g (10,9 mMol) N-Trifluoracetyl-4-acetylamino-3-nitro-phenylalanyl-(4-methyl-piperidin) in 10 ml Ethanol werden bei 20°C 28,0 ml 1N Natronlauge getropft. Nach 3-stündigem Rühren bei 50°C wird abgekühlt, nach weiteren 2 Stunden abgesaugt und mit wenig Eiswasser gewaschen.
Ausbeute: 3,1 g (93 % der Theorie),
Schmelzpunkt: 160-161°C

| C₁₅H₂₂N₄O₃ (306,36) | | | |
|---|---|---|---|
| Ber.: | C 58,81 | H 7,21 | N 18,29 |
| Gef.: | 58,63 | 7,03 | 18,34 |

### Beispiel VI

### 4-Amino-N-[1-(4-amino-3-nitro-phenyl-methyl)-2-(4-methyl-piperidin-1-yl)-2-oxo-ethyl]-3,5-dichlor-benzolsulfonamid

3,06 g (10 mMol) 4-Amino-3-nitro-phenylalanyl-(4-methyl-piperidin) werden in 100 ml Methylenchlorid gelöst und mit 2,8 ml (20 mMol) Triethylamin versetzt. Dann wird bei Raumtemperatur 3,1 g (12 mMol) 4-Amino-3,5-dichlor-benzolsulfonsäurechlorid in 20 ml Methylenchlorid langsam zugetropft. Nach 2 Stunden wird der Niederschlag abgesaugt, mit Methylenchlorid gewaschen und bei 70°C getrocknet. Zur weiteren Reinigung wird der Niederschlag in 100 ml Ethanol suspendiert, aufgekocht und nach dem Abkühlen wieder abgesaugt.
Ausbeute: 4,6 g (86,8 % der Theorie),
Schmelzpunkt: 236-237°C

| C₂₁H₂₅Cl₂N₅O₅S (530,43) | | | |
|---|---|---|---|
| Ber.: | C 47,55 | H 4,75 | N 13,20 |
| Gef.: | 47,55 | 4,82 | 13,40 |

Analog werden folgende Verbindungen erhalten:
N-[1-(4-Amino-3-nitro-phenyl-methyl)-2-(4-methyl-piperidin-1-yl)-2-oxo-ethyl]-2,5-dimethyl-benzolsulfonamid
N-[1-(4-Amino-3-nitro-phenyl-methyl)-2-(4-methyl-piperidin-1-yl)-2-oxo-ethyl]-6,7-dimethoxy-naphthalin-2-sulfonamid
N-[1-(4-Amino-3-nitro-phenyl-methyl)-2-(4-methyl-piperidin-1-yl)-2-oxo-ethyl]-5-dimethylamino-naphthalin-1-sulfonamid
N-[1-(4-Amino-3-nitro-phenyl-methyl)-2-(4-methyl-piperidin-1-yl)-2-oxo-ethyl]-2'-fluor-4-biphenylyl-sulfonamid
4-Amino-N-[1-(4-amino-3-nitro-phenyl-methyl)-2-(4,4-ethylendioxy-piperidin-1-yl)-2-oxo-ethyl]-3,5-dichlor-benzolsulfonamid
4-Amino-N-[1-(4-amino-3-nitro-phenyl-methyl)-2-(5,6,7,8-tetrahydro-4H-thiazolo[5,4-d]azepin-6-yl)-2-oxo-ethyl]-3,5-dichlor-benzolsulfonamid
4-Amino-N-[1-(4-amino-3-nitro-phenyl-methyl)-2-(5,6,7,8-tetrahydro-4H-thieno[2,3-d]azepin-6-yl)-2-oxo-ethyl]-3,5-dichlor-benzolsulfonamid
4-Amino-N-[1-(4-amino-3-nitro-phenyl-methyl)-2-(4,4-ethylen-dioxy-piperidin-1-yl)-2-oxo-ethyl]-3,5-dichlor-benzolsulfonamid
4-Amino-N-[1-(4-amino-3-nitro-phenyl-methyl)-2-(1-oxido-thiomorpholin-4-yl)-2-oxo-ethyl]-3,5-dichlor-benzolsulfonamid
4-Amino-N-[1-(4-amino-3-nitro-phenyl-methyl)-2-(4-n-propyl-piperidin-1-yl)-2-oxo-ethyl]-3,5-dichlor-benzolsulfonamid
N-[1-(4-Amino-3-nitro-phenyl-methyl)-2-(4-methyl-piperidin-1-yl)-2-oxo-ethyl]-naphthalin-2-sulfonamid
N-[1-(4-Amino-3-nitro-phenyl-methyl)-2-(4-methyl-piperidin-1-yl)-2-oxo-ethyl]-4-biphenylyl-sulfonamid
N-[1-(4-Amino-3-nitro-phenyl-methyl)-2-(4-methyl-piperidin-1-yl)-2-oxo-ethyl]-3'-nitro-4-biphenylyl-sulfonamid
4-Cyclohexyl-N-[1-(4-amino-3-nitro-phenyl-methyl)-2-(4-methyl-piperidin-1-yl)-2-oxo-ethyl]-benzolsulfonamid

### Beispiel VII

### 1-(1H-Benzimidazol-5-yl-methyl)-2-[(4-methyl-piperidin-1-yl)]-2-oxo-ethylamin

20 g (0,065 Mol) 4-Amino-3-nitro-phenylalanyl-(4-methyl-piperidin) werden in 250 ml Ameisensäure gelöst, mit 2,0 g Palladium/Kohle versetzt und im Autoklaven 1 Stunde bei 5 bar und 25°C hydriert. Anschließend wird vom Katalysator abfiltriert und das Filtrat im Vakuum einrotiert und der Rückstand in 50 ml Wasser aufgenommen. Dann wird mit 6N Natronlauge alkalisch gemacht und mit Methylenchlorid extrahiert. Die vereinigten organischen Extrakte werden über Natriumsulfat getrocknet und einrotiert.
Ausbeute: 15,2 g (82 % der Theorie),
Schmelzpunkt: 160-162°C

| C₁₆H₂₂N₄O x 0,6 H₂O (297,15) | | | |
|---|---|---|---|
| Ber. x 0,6 H₂O: | C 64,67 | H 7,87 | N 18,85 |
| Gef.: | 64,88 | 7,76 | 18,92 |

### Beispiel VIII

### N-(4-Amino-3,5-dichlor-benzolsulfamoyl)-4-amino-3-nitro-phenylalanin

10 g (0,0275 Mol) N-Trifluoracetyl-4-acetylamino-3-nitro-phenylalanin werden in 20 ml Ethanol gelöst und mit 82 ml 1N Natronlauge bei Raumtemperatur 3 Stunden gerührt. Anschließend wird eine Lösung von 10,7 g (0,041 Mol) 4-Amino-3,5-dichlor-benzolsulfonsäurechlorid, gelöst in 30 ml Aceton zugetropft, wobei der pH-Wert durch portionsweise Zugabe von Natronlauge bei einem pH Wert von etwa 10,7 gehalten wird. Nach 12-stündigem Rühren bei Raumtemperatur wird das organische Lösungsmittel im Vakuum entfernt und die wässrige Lösung auf pH 4,5 angesäuert. Der ausgefallene Niederschlag wird nach 2-stündigem Rühren abgesaugt, mit Wasser gewaschen und getrocknet.
Ausbeute: 10,7 g (86,6 % der Theorie),
Schmelzpunkt: 204-208°C (Zers.)

| C₁₅H₁₄Cl₂N₄O₆S (449,26) | | | | | |
|---|---|---|---|---|---|
| Ber.: | C 40,10 | H 3,14 | N 12,47 | S 7,13 | Cl 15,78 |
| Gef.: | 39,79 | 3,03 | 12,58 | 7,44 | 16,38 |

### Beispiel IX

### 2-(4-Amino-3,5-dichlor-benzolsulfamoyl)-3-(1H-benzimidazol-5-yl)-propionsäure

Zu einer Mischung von 47,0 g (0,105 Mol) N-(4-Amino-3,5-dichlor-benzolsulfonyl)-4-amino-3-nitro-phenylalanin und 1000 ml Ameisensäure werden 15 g Palladium/Kohle gegeben. Man hydriert die so erhaltene Reaktionsmischung 3,5 Stunden lang bei 50-60°C und einem Wasserstoffdruck von 5 bar. Anschließend wird der Katalysator abgesaugt und das Filtrat im Vakuum eingeengt. Das erhaltene Produkt wird zur weiteren Reinigung mit 40 ml Thionylchlorid, gelöst in 800 ml Ethanol, verestert und anschließend über eine Kieselgelsäule (Laufmittel: Essigester/Methanol/Ammoniak = 9:1:0,1) chromatographiert. 10,5 g des so erhaltenen Esters werden in 150 ml Ethanol gelöst und mittels 80 ml 1N Natronlauge verseift und anschließend das gewünschte Produkt durch Neutralisierung mit Wasser/Salzsäure ausgefällt und mit Ethanol nachgewaschen.
Ausbeute: 9,6 g (97 % der Theorie),
Schmelzpunkt: ab 190°C (Zers.)

| C₁₆H₁₄Cl₂N₄O₄S (429,28) | | | | | |
|---|---|---|---|---|---|
| Ber.: | C 44,77 | H 3,29 | N 13,05 | S 7,47 | Cl 16,52 |
| Gef.: | 44,97 | 3,22 | 12,85 | 7,40 | 16,00 |

### Herstellung der Endprodukte:

### Beispiel 1

### 4-Amino-N-[1-(1H-benzimidazol-5-yl-methyl)-2-(4-methyl-piperidin-1-yl)-2-oxo-ethyl]-3,5-dichlor-benzolsulfonamid

1,06 g (2 mMol) 4-Amino-N-[1-(4-amino-3-nitro-phenylmethyl)-2-(4-methyl-piperidin-1-yl)-2-oxo-ethyl]-3,5-dichlor-benzolsulfonamid werden in 20 ml Ameisensäure suspendiert und unter Zusatz von 0,1 g Palladium/Kohle im Autoklaven bei einem Wasserstoffdruck von 5 bar 80 Minuten bei Raumtemperatur hydriert. Danach wird vom Katalysator abfiltriert und das Filtrat 2 Stunden auf 50°C erwärmt. Anschließend wird eingeengt, mit 30 ml Wasser versetzt, mit konzentriertem Ammoniak alkalisch gestellt und 2 x mit Essigester extrahiert. Die organische Phase wird 2 x mit Wasser gewaschen, über Magnesiumsulfat getrocknet und eingedampft. Der erhaltene Rückstand wird auf einer Kieselgelsäule (Essigester/Methanol/Ammoniak = 95:5:0,5) gereinigt. Nach Eindampfen wird aus Isopropanol umkristallisiert.
Ausbeute: 600 mg (59 % der Theorie),
Schmelzpunkt: 233-235°C

| C₂₂H₂₅Cl₂N₅O₃S (510,45) | | | | | |
|---|---|---|---|---|---|
| Ber.: | C 51,87 | H 4,75 | N 13,75 | S 6,29 | Cl 13,92 |
| Gef.: | 51,61 | 4,93 | 13,39 | 6,27 | 14,43 |

### Beispiel 2

### N-[1-(1H-Benzimidazol-5-yl-methyl)-2-(4-methyl-piperidin-1-yl)-2-oxo-ethyl]-9-methyl-1,2,3,4-tetrahydro-carbazol-6-sulfonamid

Zu einer Lösung von 1 g (0,0034 Mol) 1-(1H-Benzimidazol-5-yl-methyl)-2-(4-methyl-piperidin-1-yl)-2-oxo-ethylamin und 1,1 g (0,011 Mol) Triethylamin in 30 ml Chloroform werden bei Raumtemperatur unter Rühren 0,96 g (0,0034 Mol) 9-Methyl-1,2,3,4-tetrahydro-carbazol-6-sulfonsäurechlorid, gelöst in 10 ml Chloroform, zugetropft. Nach 12-stündigem Rühren wird im Vakuum eingeengt und der erhaltene Rückstand über eine Kieselgelsäule (Laufmittel: Ethylacetat/Methanol = 9:1) chromatographiert. Das Eluat mit einem R_{f}-Wert von 0,3 wird gesammelt und im Vakuum eingeengt.
Ausbeute: 0,9 g (50 % der Theorie),
Schmelzpunkt: 190-192°C (Zers.)

| C₂₉H₃₅N₅O₃S (533,67) | | | | |
|---|---|---|---|---|
| Ber.: | C 65,26 | H 6,60 | N 13,12 | S 6,00 |
| Gef.: | 64,96 | 6,64 | 12,94 | 5,97 |

### Beispiel 3

### N-[1-(1H-Benzimidazol-5-yl-methyl)-2-(4-methyl-piperidin-1-yl)-2-oxo-ethyl]-2,5-dimethyl-benzolsulfonamid

Hergestellt aus 4-Amino-N-[1-(4-amino-3-nitro-phenylmethyl)-2-(4-methyl-piperidin-1-yl)-2-oxo-ethyl]-3,5-dimethyl-benzolsulfonamid analog Beispiel 1.
Ausbeute: 64 % der Theorie,
Schmelzpunkt: 144-146°C

| C₂₄H₃₀N₄O₃S x H₂O (472,60) | | | |
|---|---|---|---|
| Ber.: | C 60,99 | H 6,83 | N 11,87 |
| Gef.: | 61,22 | 6,97 | 11,43 |

### Beispiel 4

### N-[1-(1H-Benzimidazol-5-yl-methyl)-2-(4-methyl-piperidin-1-yl)-2-oxo-ethyl]-6,7-dimethoxy-naphthalin-2-sulfonamid

Hergestellt aus 4-Amino-N-[1-(4-amino-3-nitro-phenylmethyl)-2-(4-methyl-piperidin-1-yl)-2-oxo-ethyl]-6,7-dimethoxy-naphthalin-2-sulfonamid analog Beispiel 1.
Ausbeute: 36,6 % der Theorie,
Schmelzpunkt: Schaum

| C₂₈H₃₂N₄O₅S (536,66) | | | | |
|---|---|---|---|---|
| Ber.: | C 62,67 | H 6,01 | N 10,44 | S 5,97 |
| Gef.: | 62,40 | 6,17 | 10,05 | 5,54 |

### Beispiel 5

### N-[1-(1H-Benzimidazol-5-yl-methyl)-2-(4-methyl-piperidin-1-yl)-2-oxo-ethyl]-5-dimethylamino-naphthalin-1-sulfonamid

Hergestellt aus 4-Amino-N-[1-(4-amino-3-nitro-phenylmethyl)-2-(4-methyl-piperidin-1-yl)-2-oxo-ethyl]-5-dimethylamino-naphthalin-1-sulfonamid analog Beispiel 1.
Ausbeute: 66,8 % der Theorie,
Schmelzpunkt: ab 210°C (Zers.)

| C₂₈H₃₃N₅O₃S (519,67) | | | | |
|---|---|---|---|---|
| Ber.: C | 64,72 | H 6,40 | N 13,48 | S 6,17 |
| Gef.: | 64,56 | 6,37 | 13,69 | 5,96 |

### Beispiel 6

### N-[1-(1H-Benzimidazol-5-yl-methyl)-2-(4-ethyl-piperidin-1-yl)-2-oxo-ethyl]-3,5-dichlor-4-dimethylamino-benzolsulfonamid

Zu einer auf 110°C erhitzten Mischung aus 0,7 g (0,0078 Mol) Paraformaldehyd und 20 ml Ameisensäure werden portionsweise 1,6 g (0,003 Mol) 4-Amino-N-[1-(1H-benzimidazol-5-yl-methyl)-2-(4-ethyl-piperidin-1-yl)-2-oxo-ethyl]-3,5-dichlor-benzolsulfonamid eingetragen. Anschließend wird eine weitere Stunde auf 120°C erhitzt. Es wird abgekühlt, im Vakuum eingeengt und der erhaltene Rückstand über eine Kieselgelsäule (Laufmittel: Essigester/Methanol = 19:1) chromatographiert. Die Fraktionen mit einem R_{f}-Wert von 0,43 werden gesammelt und im Vakuum eingeengt.
Ausbeute: 19,3 % der Theorie,
Schmelzpunkt: ab 86°C (Zers.)

| C₂₅H₃₁Cl₂N₅O₃S (552,53) | | | | |
|---|---|---|---|---|
| Ber. x H₂O: | C 52,62 | H 5,62 | N 12,27 | S 5,61 |
| Gef.: | 52,46 | 5,45 | 12,23 | 5,64 |

### Beispiel 7

### N-[1-(1H-Benzimidazol-5-yl-methyl)-2-(4-methyl-piperidin-1-yl)-2-oxo-ethyl]-3,5-dichlor-4-dimethylamino-benzolsulfonamid

Hergestellt aus 4-Amino-N-[1-(1H-benzimidazol-5-yl-methyl)-2-(4-methyl-piperidin-1-yl)-2-oxo-ethyl]-3,5-dichlor-benzolsulfonamid und Paraformaldehyd analog Beispiel 6.
Ausbeute: 18,0 % der Theorie,
Schmelzpunkt: ab 100°C (Zers.)

| C₂₄H₂₉Cl₂N₅O₃S (538,50) | | | | | |
|---|---|---|---|---|---|
| Ber.: | C 53,53 | H 5,42 | N 13,00 | S 5,95 | Cl 13,16 |
| Gef.: | 53,77 | 5,38 | 12,85 | 6,03 | 13,74 |

### Beispiel 8

### N-[1-(1H-Benzimidazol-5-yl-methyl)-2-(4-ethyl-piperidin-1-yl)-2-oxo-ethyl]-3,5-dichlor-4-ethylamino-benzolsulfonamid

Hergestellt aus 4-Amino-N-[1-(1H-benzimidazol-5-yl-methyl)-2-(4-methyl-piperidin-1-yl)-2-oxo-ethyl]-3,5-dichlor-benzolsulfonamid und Acetaldehyd analog Beispiel 6.
Ausbeute: 8,6 % der Theorie,
Schmelzpunkt: ab 140°C (Zers.)

| C₂₅H₃₁Cl₂N₅O₃S (552,53) | | | |
|---|---|---|---|
| Ber.: | C 54,34 | H 5,65 | N 12,67 |
| Gef.: | 54,09 | 5,46 | 12,44 |

### Beispiel 9

### N-[1-(1H-Benzimidazol-5-yl-methyl)-2-(4-methyl-piperidin-1-yl)-2-oxo-ethyl]-3,5-dichlor-4-pyrrolyl-benzolsulfonamid

1,1 g (0,00215 Mol) 4-Amino-N-[1-(1H-benzimidazol-5-yl-methyl)-2-(4-methyl-piperidin-1-yl)-2-oxo-ethyl]-3,5-dichlor-benzolsulfonamid werden mit 0,4 g (0,03 Mol) 2,5-Dimethoxytetrahydrofuran in 3 ml Eisessig und 3 ml Methanol 4 Stunden unter Rückfluß erhitzt. Anschließend wird im Vakuum zur Trockene eingeengt und der erhaltene Rückstand über eine Kieselgelsäule (Laufmittel: Ethylacetat/Methanol = 9:1) chromatographiert. Die Fraktionen mit einem R_{f}-Wert von 0,4 werden gesammelt und im Vakuum eingeengt.
Ausbeute: 25,0 % der Theorie,
Schmelzpunkt: ab 100°C (Zers.)

| C₂₆H₂₇Cl₂N₅O₃S (560,51) | | | | | |
|---|---|---|---|---|---|
| Ber.: | C 55,71 | H 4,85 | N 12,49 | S 5,71 | Cl 12,65 |
| Gef.: | 55,95 | 5,12 | 11,91 | 5,76 | 12,48 |

### Beispiel 10

### N-[1-(1H-Benzimidazol-5-yl-methyl)-2-(4-methyl-piperidin-1-yl)-2-oxo-ethyl]-2'-fluor-4-biphenylyl-sulfonamid

Hergestellt aus 4-Amino-N-[1-(4-amino-3-nitro-phenylmethyl)-2-(4-methyl-piperidin-1-yl)-2-oxo-ethyl]-2'-fluor-4-biphenylyl-sulfonamid analog Beispiel 1.
Ausbeute: 67,0 % der Theorie,
Schmelzpunkt: ab 195°C (Zers.)

| C₂₈H₂₉FN₄O₃S (520,63) | | | | |
|---|---|---|---|---|
| Ber.: | C 64,60 | H 5,61 N | 10,76 | S 6,16 |
| Gef.: | 64,20 | 5,44 | 11,04 | 6,28 |

### Beispiel 11

### N-[1-(1H-Benzimidazol-5-yl-methyl)-2-(4-methyl-piperidin-1-yl)-2-oxo-ethyl]-dibenzofuran-2-sulfonamid

Hergestellt aus 1-(1H-Benzimidazol-5-yl-methyl)-2-(4-methyl-piperidin-1-yl)-2-oxo-ethylamin und Dibenzofuran-2-sulfonsäurechlorid analog Beispiel 2.
Ausbeute: 39,0 % der Theorie,
Schmelzpunkt: ab 85°C (Zers.)

| C₂₈H₂₈N₄O₄S (516,62) | | | | |
|---|---|---|---|---|
| Ber. x H₂O: | C 62,90 | H 5,65 | N 10,48 | S 5,99 |
| Gef.: | 62,70 | 5,82 | 9,94 | 6,37 |

### Beispiel 12

### N-[1-(1H-Benzimidazol-5-yl-methyl)-2-(4-methyl-piperidin-1-yl)-2-oxo-ethyl]-9-ethyl-carbazol-3-sulfonamid

Hergestellt aus 1-(1H-Benzimidazol-5-yl-methyl)-2-(4-methyl-piperidin-1-yl)-2-oxo-ethylamin und 9-Ethyl-carbazol-3-sulfonsäurechlorid analog Beispiel 2.
Ausbeute: 54,0 % der Theorie,
Schmelzpunkt: ab 100°C (Zers.)

| C₃₀H₃₃N₅O₃S (543,69) | | | | |
|---|---|---|---|---|
| Ber. x H₂O: | C 64,15 | H 6,27 | N 12,46 | S 5,70 |
| Gef.: | 64,04 | 6,10 | 11,60 | 5,76 |

### Beispiel 13

### 3-[[1-(1H-Benzimidazol-5-yl-methyl)-2-(4-methyl-piperidin-1-yl)-2-oxo-ethyl]sulfamoyl]-carbazol-9-yl-essigsäure-ethylester

Hergestellt aus 1-(1H-Benzimidazol-5-yl-methyl)-2-(4-methyl-piperidin-1-yl)-2-oxo-ethylamin und 9-Ethoxycarbonylmethylcarbazol-3-sulfonsäurechlorid analog Beispiel 2.
Ausbeute: 56,0 % der Theorie,
Schmelzpunkt: ab 110°C (Zers.)

| C₃₂H₃₅N₅O₅S (601,73) | | | | |
|---|---|---|---|---|
| Ber.: | C 63,87 | H 5,86 | N 11,63 | S 5,32 |
| Gef.: | 63,62 | 6,04 | 11,42 | 5,62 |

### Beispiel 14

### N-[1-(1H-Benzimidazol-5-yl-methyl)-2-(4-methyl-piperidin-1-yl)-2-oxo-ethyl]-8-hydroxy-naphthalin-1-sulfonamid

Hergestellt aus 1-(1H-Benzimidazol-5-yl-methyl)-2-(4-methyl-piperidin-1-yl)-2-oxo-ethylamin und 1,8-Naphthalinsulton durch 30-minütiges Erhitzen auf 100°C und anschließendes 20-minütiges Erhitzen auf 140°C. Nach dem Abkühlen wird das erhaltene Produkt über eine Kieselgelsäule (Laufmittel: Essigester/Methanol = 9:1) gereinigt.
Ausbeute: 20,0 % der Theorie,
Schmelzpunkt: 240-242°C

| C₂₆H₂₈N₄O₄S (492,60) | | | | |
|---|---|---|---|---|
| Ber.: | C 63,39 | H 5,72 | N 11,37 | S 6,50 |
| Gef.: | 63,27 | 5,64 | 11,19 | 6,33 |

### Beispiel 15

### 4-Amino-N-[1-(1H-benzimidazol-5-yl-methyl)-2-(4-ethyl-piperidin-1-yl)-2-oxo-ethyl]-3,5-dichlor-benzolsulfonamid

1,4 g (0,003 Mol) 2-(4-Amino-3,5-dichlor-benzolsulfamoyl)-3-(1H-benzimidazol-5-yl)-propionsäure, 0,45 g (0,003 Mol) 4-Ethyl-piperidin-hydrochlorid, 0,405 g (0,003 Mol) 1-Hydroxy-1H-benztriazol und 1 g (0,009 Mol) N-Ethyl-morpholin werden in 15 ml Dimethylformamid gelöst, auf 0°C gekühlt und schließlich mit 0,72 g (0,0035 Mol) N,N'-Dicyclohexylcarbodiimid versetzt. Nach 18-stündigem Rühren wird der ausgefallene Dicyclohexylharnstoff abgesaugt und das Filtrat im Vakuum eingeengt. Der erhaltene Rückstand wird über eine Kieselgelsäule (Laufmittel: Methylenchlorid/Ethanol = 9:1) chromatographiert und die Fraktionen mit einem R_{f}-Wert von 0,68 gesammelt und im Vakuum eingeengt.
Ausbeute: 70,0 % der Theorie,
Schmelzpunkt: ab 95°C (Zers.)

| C₂₃H₂₇Cl₂N₅O₃S (524,47) | | | | | |
|---|---|---|---|---|---|
| Ber.: | C 52,67 | H 5,18 N | 13,35 | S 6,11 | Cl 13,52 |
| Gef.: | 52,68 | 5,23 | 12,90 | 6,04 | 13,40 |

### Beispiel 16

### 4-Amino-N-[1-(1H-benzimidazol-5-yl-methyl)-2-(2,4,6-trimethyl-piperidin-1-yl)-2-oxo-ethyl]-3,5-dichlor-benzolsulfonamid

Hergestellt aus 2-(4-Amino-3,5-dichlor-benzolsulfamoyl)-3-(1H-benzimidazol-5-yl)-propionsäure und 2,4,6-Trimethyl-piperidin analog Beispiel 15.
Ausbeute: 10,0 % der Theorie,
Schmelzpunkt: ab 90°C (Zers.)

| C₂₄H₂₉Cl₂N₅O₃S (538,50) | | | | | |
|---|---|---|---|---|---|
| Ber.: | C 53,53 | H 5,42 | N 13,00 | S 5,95 | Cl 13,16 |
| Gef.: | 53,16 | 5,69 | 12,86 | 6,12 | 12,95 |

### Beispiel 17

### 4-Amino-N-[1-(1H-Benzimidazol-5-yl-methyl)-2-(N-benzyl-methylamino)-2-oxo-ethyl]-3,5-dichlor-benzolsulfonamid

Hergestellt aus 2-(4-Amino-3,5-dichlor-benzolsulfamoyl)-3-(1H-benzimidazol-5-yl)-propionsäure und N-Benzyl-methylamin analog Beispiel 15.
Ausbeute: 10,0 % der Theorie,
Schmelzpunkt: ab 80°C (Zers.)

| C₂₄H₂₃Cl₂N₅O₃S (532,45) | | | | | |
|---|---|---|---|---|---|
| Ber.: | C 54,13 | H 4,35 | N 13,15 | S 6,02 | Cl 13,31 |
| Gef.: | 54,45 | 4,27 | 13,08 | 5,86 | 12,28 |

### Beispiel 18

### 4-Amino-N-[1-(1H-benzimidazol-5-yl-methyl)-2-(piperidin-1-yl)-2-oxo-ethyl]-3,5-dichlor-benzolsulfonamid

Hergestellt aus 2-(4-Amino-3,5-dichlor-benzolsulfamoyl)-3-(1H-benzimidazol-5-yl)-propionsäure und Piperidin analog Beispiel 15.
Ausbeute: 67,0 % der Theorie,
Schmelzpunkt: 272-274°C (sintern ab 245°C)

| C₂₁H₂₃Cl₂N₅O₃S (496,42) | | | | | |
|---|---|---|---|---|---|
| Ber.: | C 50,81 | H 4,66 | N 14,10 | S 6,45 | Cl 14,28 |
| Gef.: | 50,66 | 4,52 | 14,09 | 6,60 | 14,18 |

### Beispiel 19

### 4-Amino-N-[1-(1H-benzimidazol-5-yl-methyl)-2-(N-methyl-indan-1-yl-amino)-2-oxo-ethyl]-3,5-dichlor-benzolsulfonamid

Hergestellt aus 2-(4-Amino-3,5-dichlor-benzolsulfamoyl)-3-(1H-benzimidazol-5-yl)-propionsäure und N-Methyl-indan-1-yl-amin analog Beispiel 15.
Ausbeute: 23,0 % der Theorie,
Schmelzpunkt: 156°C

| C₂₆H₂₅Cl₂N₅O₃S (558,49) | | | |
|---|---|---|---|
| Ber.: | C 55,92 | H 4,51 | N 12,54 |
| Gef.: | 56,08 | 4,63 | 12,84 |

### Beispiel 20

### N-[1-(1H-Benzimidazol-5-yl-methyl)-2-(4-methyl-piperidin-1-yl)-2-oxo-ethyl]-4-chlor-3-nitro-benzolsulfonamid

Hergestellt aus 1-(1H-Benzimidazol-5-yl-methyl)-2-(4-methyl-piperidin-1-yl)-2-oxo-ethylamin und 4-Chlor-3-nitro-benzolsulfonsäurechlorid analog Beispiel 2.
Ausbeute: 31,0 % der Theorie,
Schmelzpunkt: 110°C (Zers.)

| C₂₂H₂₄ClN₅O₅S (505,98) | | | | | |
|---|---|---|---|---|---|
| Ber.: | C 52,22 | H 4,78 | N 13,84 | S 6,33 | Cl 7,00 |
| Gef.: | 52,22 | 4,99 | 13,56 | 6,20 | 7,08 |

### Beispiel 21

### N-[1-(1H-Benzimidazol-5-yl-methyl)-2-(4-methyl-piperidin-1-yl)-2-oxo-ethyl]-2-nitro-benzolsulfonamid

Hergestellt aus 1-(1H-Benzimidazol-5-yl-methyl)-2-(4-methyl-piperidin-1-yl)-2-oxo-ethylamin und 2-Nitro-benzolsulfonsäure-chlorid analog Beispiel 2.
Ausbeute: 57,0 % der Theorie,
Schmelzpunkt: 125°C

| C₂₂H₂₅N₅O₅S (471,54) | | | |
|---|---|---|---|
| Ber.: | C 56,04 | H 5,34 | N 14,85 |
| Gef.: | 56,19 | 5,56 | 14,39 |

### Beispiel 22

### 3-Amino-N-[1-(1H-benzimidazol-5-yl-methyl)-2-(4-methyl-piperidin-1-yl)-2-oxo-ethyl]-4-chlor-benzolsulfonamid

5 g (0,01 Mol) N-[1-(1H-Benzimidazol-5-yl-methyl)-2-(4-methyl-piperidin-1-yl)-2-oxo-ethyl]-4-chlor-3-nitro-benzolsulfonamid werden in 50 ml Methanol gelöst und in Gegenwart von 1 g Platin/Kohle und einem Wasserstoffdruck von 3 bar bei Raumtemperatur hydriert. Anschließend wird vom Katalysator abgesaugt, das Filtrat eingeengt, der Rückstand mit Ether verrieben und abgesaugt.
Ausbeute: 4,5 g (94,5 % der Theorie),
Schmelzpunkt: ab 140°C (Zers.)

| C₂₂H₂₆ClN₅O₃S (467,00) | | | | |
|---|---|---|---|---|
| Ber. x H₂O: | C 53,48 | H 5,71 | N 14,71 | S 6,48 |
| Gef.: | 53,60 | 5,46 | 14,48 | 6,45 |

### Beispiel 23

### 2-Amino-N-[1-(1H-benzimidazol-5-yl-methyl)-2-(4-methyl-piperidin-1-yl)-2-oxo-ethyl]-benzolsulfonamid

Hergestellt aus N-[1-(1H-Benzimidazol-5-yl-methyl)-2-(4-methyl-piperidin-1-yl)-2-oxo-ethyl]-2-nitro-benzolsulfonamid durch katalytische Hydrierung analog Beispiel 22.
Ausbeute: 29,5 % der Theorie,
Schmelzpunkt: ab 126°C (Zers.)

| C₂₂H₂₇N₅O₃S (441,56) | | | |
|---|---|---|---|
| Ber.: | C 59,84 | H 6,16 | N 15,86 |
| Gef.: | 58,18 | 6,36 | 15,61 |

### Beispiel 24

### 3-[[1-(1H-Benzimidazol-5-yl-methyl)-2-(4-methyl-piperidin-1-yl)-2-oxo-ethyl]sulfamoyl]-carbazol-9-yl-essigsäure

Zu einer Lösung von 0,6 g (0,001 Mol) 3-[[1-(1H-Benzimidazol-5-yl-methyl)-2-(4-methyl-piperidin-1-yl)-2-oxo-ethyl]sulfamoyl]-carbazol-9-yl-essigsäure-ethylester in 5 ml Dioxan und 5 ml Methanol werden 4 ml 1N Natronlauge getropft und eine Stunde bei 20°C gerührt. Anschließend wird mit 20 ml Wasser verdünnt und mit 4 ml 1N Salzsäure versetzt. Das organische Lösungsmittel wird im Vakuum entfernt, das ausgefallene Produkt abgesaugt, mit Wasser gewaschen und getrocknet.
Ausbeute: 0,5 g (87,0 % der Theorie),
Schmelzpunkt: 220°C (Zers.)

| C₃₀H₃₁N₅O₅S (573,68) | | | | |
|---|---|---|---|---|
| Ber.: | C 62,81 | H 5,44 | N 12,20 | S 5,58 |
| Gef.: | 62,66 | 5,57 | 11,98 | 5,24 |

### Beispiel 25

### 4-Amino-N-[1-(1H-benzimidazol-5-yl-methyl)-2-(piperidin-4-on-1-yl)-2-oxo-ethyl]-3,5-dichlor-benzolsulfonamid

Hergestellt aus 4-Amino-N-[1-(4-amino-3-nitro-phenyl-methyl]-2-(piperidin-4,4-ethylendioxy-1-yl)-2-oxo-ethyl]-3,5-dichlor-benzolsulfonamid und Cyclisierung mit Ameisensäure in Gegenwart von Palladium/Kohle analog Beispiel 1.
Ausbeute: 81 % der Theorie,
Schmelzpunkt: 235-237°C (Zers.)

| C₂₁H₂₁Cl₂N₅O₄S (510,40) | | | | | |
|---|---|---|---|---|---|
| Ber.: | C 49,42 | H 4,15 | N 13,72 | S 6,28 | Cl 13,89 |
| Gef.: | 49,59 | 4,42 | 13,44 | 6,44 | 14,07 |

### Beispiel 26

### 4-Amino-N-[1-(1H-benzimidazol-5-yl-methyl)-2-(5,6,7,8-tetrahydro-4H-thiazolo[4,5-d]azepin-6-yl)-2-oxo-ethyl]-3,5-dichlor-benzolsulfonamid

Hergestellt aus 4-Amino-N-[1-(4-amino-3-nitro-phenyl-methyl)-2-(5,6,7,8-tetrahydro-4H-thiazolo[4,5-d]azepin-6-yl)-2-oxo-ethyl]-3,5-dichlor-benzolsulfonamid und Cyclisierung mit Ameisensäure in Gegenwart von Palladium/Kohle analog Beispiel 1.
Ausbeute: 44 % der Theorie,
Schmelzpunkt: 156-159°C

| C₂₃H₂₂Cl₂N₆O₃S₂ (565,50) | | | | | |
|---|---|---|---|---|---|
| Ber.: C | 48,85 | H 3,92 | N 14,86 S | 11,34 | Cl 12,54 |
| Gef.: | 48,66 | 4,38 | 14,58 | 11,54 | 12,67 |

### Beispiel 27

### 4-Amino-N-[1-(1H-benzimidazol-5-yl-methyl)-2-(5,6,7,8-tetrahydro-4H-thieno[2,3-d]azepin-6-yl)-2-oxo-ethyl]-3,5-dichlor-benzolsulfonamid

Hergestellt aus 4-Amino-N-[1-(4-amino-3-nitro-phenyl-methyl)-2-(5,6,7,8-tetrahydro-4H-thieno[2,3-d]azepin-6-yl)-2-oxo-ethyl]-3,5-dichlor-benzolsulfonamid und Cyclisierung mit Ameisensäure in Gegenwart von Palladium/Kohle analog Beispiel 1.
Ausbeute: 50 % der Theorie,
Schmelzpunkt: 143-146°C

| C₂₄H₂₃Cl₂N₅O₃S₂ (564,52) | | | | | |
|---|---|---|---|---|---|
| Ber.: | C 51,07 | H 4,11 | N 12,41 | S 11,36 | Cl 12,56 |
| Gef.: | 50,92 | 4,47 | 12,46 | 11,13 | 12,88 |

### Beispiel 28

### N-[1-(1H-Benzimidazol-5-yl-methyl)-2-(4-methyl-piperidin-1-yl)-2-oxo-ethyl]-3,5-di-tert.butyl-4-hydroxy-benzolsulfonamid

Hergestellt aus N-[1-(4-Amino-3-nitro-phenyl-methyl)-2-(4-methyl-piperidin-1-yl)-2-oxo-ethyl]-3,5-di-tert.butyl-4-hydroxy-benzolsulfonamid und Cyclisierung mit Ameisensäure in Gegenwart von Palladium/Kohle analog Beispiel 1.
Ausbeute: 48,6 % der Theorie,
Schmelzpunkt: 200-202°C

| C₃₀H₄₂N₄O₄S (554,76) | | | | |
|---|---|---|---|---|
| Ber.: | C 64,95 | H 7,63 | N 10,10 | S 5,78 |
| Gef.: | 64,63 | 7,96 | 9,92 | 5,63 |

### Beispiel 29

### 4-Amino-N-[1-(1H-benzimidazol-5-yl-methyl)-2-(1-oxido-thiomorpholin-4-yl)-2-oxo-ethyl]-3,5-dichlor-benzolsulfonamid

Hergestellt aus 4-Amino-N-[1-(4-amino-3-nitro-phenyl-methyl)-2-(1-oxido-thiomorpholin-4-yl)-2-oxo-ethyl]-3,5-dichlor-benzolsulfonamid und Cyclisierung mit Ameisensäure in Gegenwart von Palladium/Kohle analog Beispiel 1.
Ausbeute: 58 % der Theorie,
Schmelzpunkt: 260-263°C

| C₂₀H₂₁Cl₂N₅O₄S₂ (530,46) | | | | | |
|---|---|---|---|---|---|
| Ber.: | C 45,29 | H 3,99 | N 13,20 | S 12,09 | Cl 13,37 |
| Gef.: | 44,98 | 4,06 | 12,99 | 12,00 | 13,08 |

### Beispiel 30

### 4-Amino-N-[1-(1H-benzimidazol-5-yl-methyl)-2-(4-propyl-piperidin-1-yl)-2-oxo-ethyl]-3,5-dichlor-benzolsulfonamid

Hergestellt aus 4-Amino-N-[1-(4-amino-3-nitro-phenyl-methyl)-2-(4-propyl-piperidin-1-yl)-2-oxo-ethyl]-3,5-dichlor-benzolsulfonamid und Cyclisierung mit Ameisensäure in Gegenwart von Palladium/Kohle analog Beispiel 1.
Ausbeute: 46 % der Theorie,
Schmelzpunkt: 173°C

| C₂₄H₂₉Cl₂N₅O₃S (538,50) | | | | | |
|---|---|---|---|---|---|
| Ber.: | C 53,53 | H 5,43 | N 13,01 | S 5,95 | Cl 13,17 |
| Gef.: | 53,47 | 5,71 | 12,60 | 6,06 | 12,74 |

### Beispiel 31

### N-[1-(1H-Benzimidazol-5-yl-methyl)-2-(4-methyl-piperidin-1-yl)-2-oxo-ethyl]-naphthalin-2-sulfonamid

Hergestellt aus N-[1-(4-Amino-3-nitro-phenyl-methyl)-2-(4-methyl-piperidin-1-yl)-2-oxo-ethyl]-naphthalin-2-sulfonamid und Cyclisierung mit Ameisensäure in Gegenwart von Palladium/Kohle analog Beispiel 1.
Ausbeute: 40 % der Theorie,
Schmelzpunkt: 130-134°C

| C₂₆H₂₈N₄O₃S (476,60) | | | | |
|---|---|---|---|---|
| Ber.: | C 65,52 | H 5,92 | N 11,76 | S 6,73 |
| Gef.: | 65,49 | 6,02 | 11,47 | 6,89 |

### Beispiel 32

### N-[1-(1H-Benzimidazol-5-yl-methyl)-2-(4-methyl-piperidin-1-yl)-2-oxo-ethyl]-4-biphenylyl-sulfonamid

Hergestellt aus N-[1-(4-Amino-3-nitro-phenyl-methyl)-2-(4-methyl-piperidin-1-yl)-2-oxo-ethyl]-4-biphenylyl-sulfonamid und Cyclisierung mit Ameisensäure in Gegenwart von Palladium/Kohle analog Beispiel 1.
Ausbeute: 63 % der Theorie,
Schmelzpunkt: 127-130°C

| C₂₈H₃₀N₄O₃S (502,64) | | | | |
|---|---|---|---|---|
| Ber.: | C 66,91 | H 6,02 | N 11,15 | S 6,38 |
| Gef.: | 66,70 | 6,09 | 11,10 | 6,39 |

### Beispiel 33

### N-[1-(1H-Benzimidazol-5-yl-methyl)-2-(4-methyl-piperidin-1-yl)-2-oxo-ethyl]-3'-amino-4-biphenylyl-sulfonamid

Hergestellt aus N-[1-(4-Amino-3-nitro-phenyl-methyl)-2-(4-methyl-piperidin-1-yl)-2-oxo-ethyl]-3'-nitro-4-biphenylyl-sulfonamid und Reduktion mit katalytisch angeregtem Wasserstoff in Gegenwart von Palladium/Kohle in Ameisensäure analog Beispiel 1.
Ausbeute: 6,3 % der Theorie,
Schmelzpunkt: ab 80°C (Zers.)

| C₂₈H₃₁N₅O₃S (517,66) | | | | |
|---|---|---|---|---|
| Ber.: | C 64,97 | H 6,04 | N 13,53 | S 6,19 |
| Gef.: | 64,75 | 6,38 | 12,37 | 6,26 |

### Beispiel 34

### 4-Cyclohexyl-N-[1-(1H-benzimidazol-5-yl-methyl)-2-(4-methyl-piperidin-1-yl)-2-oxo-ethyl]-benzolsulfonamid

Hergestellt aus 4-Cyclohexyl-N-[1-(4-amino-3-nitro-phenyl-methyl)-2-(4-methyl-piperidin-1-yl)-2-oxo-ethyl]-benzolsulfonamid und Cyclisierung mit Ameisensäure in Gegenwart von Palladium/Kohle analog Beispiel 1.
Ausbeute: 43 % der Theorie,
Schmelzpunkt: 220-225°C

| C₂₈H₃₆N₄O₃S (508,69) | | | | |
|---|---|---|---|---|
| Ber.: | C 66,12 | H 7,13 | N 11,01 | S 6,30 |
| Gef.: | 66,06 | 7,07 | 11,01 | 6,12 |

### Beispiel 35

### N-[1-(1H-Benzimidazol-5-yl-methyl)-2-(4-methyl-piperidin-1-yl)-2-oxo-ethyl]-1,2,3,4-tetrahydro-chinolin-8-sulfonamid

Hergestellt aus N-[1-(1H-Benzimidazol-5-yl-methyl)-2-(4-methyl-piperidin-1-yl)-2-oxo-ethyl]-chinolin-8-sulfonamid durch katalytische Hydrierung in Gegenwart von Palladium/Kohle in 50%iger Essigsäure analog Beispiel 1.
Ausbeute: 67 % der Theorie,
Schmelzpunkt: 126-129°C

| C₂₅H₃₁N₅O₃S (481,62) | | | | |
|---|---|---|---|---|
| Ber.: | C 62,35 | H 6,49 | N 14,54 | S 6,66 |
| Gef.: | 62,00 | 6,51 | 14,30 | 6,22 |

### Beispiel 36

### N-[1-(1H-Benzimidazol-5-yl-methyl)-2-(4-methyl-piperidin-1-yl)-2-oxo-ethyl]-chinolin-8-sulfonamid

Hergestellt aus 1-(1H-Benzimidazol-5-yl-methyl)-2-(4-methyl-piperidin-1-yl)-2-oxo-ethylamin und Chinolin-8-sulfonsäurechlorid analog Beispiel 2.
Ausbeute: 27,0 % der Theorie,
Schmelzpunkt: 123-127°C

| C₂₅H₂₇N₅O₃S (477,59) | | | | |
|---|---|---|---|---|
| Ber.: | C 62,87 | H 5,70 | N 14,66 | S 6,71 |
| Gef.: | 62,67 | 5,83 | 14,98 | 6,43 |

Analog wird folgende Verbindung erhalten:
N-[1-(1H-Benzimidazol-5-yl-methyl)-2-(4-methyl-piperidin-1-yl)-2-oxo-ethyl]-3-methyl-chinolin-8-sulfonamid

| C₂₆H₂₉N₅O₃S (491,62) | | | | |
|---|---|---|---|---|
| Ber.: | C 63,52 | H 5,95 | N 14,25 | S 6,52 |
| Gef.: | 63,30 | 6,09 | 14,00 | 6,66 |

### Beispiel 37

### N-[1-(1H-Benzimidazol-5-yl-methyl)-2-(4-methyl-piperidin-1-yl)-2-oxo-ethyl]-naphthalin-1-sulfonamid

Hergestellt aus 1-(1H-Benzimidazol-5-yl-ethyl)-2-(4-methyl-piperidin)-1-yl)-2-oxo-ethylamin und Naphthalin-1-sulfonsäurechlorid analog Beispiel 2.
Ausbeute: 38,0 % der Theorie,
Schmelzpunkt: 80-84°C

| C₂₆H₂₈N₄O₃S (476,60) | | | | |
|---|---|---|---|---|
| Ber.: | C 65,52 | H 5,92 | N 11,76 | S 6,73 |
| Gef.: | 65,76 | 6,04 | 11,52 | 6,66 |

### Beispiel 38

### N-[1-(1H-Benzimidazol-5-yl-methyl)-2-(4-methyl-piperidin-1-yl)-2-oxo-ethyl]-2'-nitro-4-biphenylyl-sulfonamid

Hergestellt aus 1-(1H-Benzimidazol-5-yl-methyl)-2-(4-methyl-piperidin)-1-yl)-2-oxo-ethylamin und 2'-Nitro-4-biphenylyl-sulfonsäurechlorid analog Beispiel 2.
Ausbeute: 38,0 % der Theorie,
Schmelzpunkt: ab 215°C

| C₂₈H₂₉N₅O₅S (547,64) | | | |
|---|---|---|---|
| Ber.: | C 61,41 | H 5,34 | N 12,79 |
| Gef.: | 61,37 | 5,38 | 12,82 |

### Beispiel 39

### 4-Amino-N-[1-(1H-benzimidazol-5-yl-methyl)-2-(4-(N-acetyl-methylamino)-piperidin-1-yl)-2-oxo-ethyl]-3,5-dichlor-benzolsulfonamid

Hergestellt aus 4-Amino-N-[1-(1H-benzimidazol-5-yl-methyl)-2-(4-methylamino-piperidin-1-yl)-2-oxo-ethyl]-3,5-dichlor-benzolsulfonamid und Acetylchlorid in Dioxan und Triethylamin analog Beispiel 2.
Ausbeute: 44,0 % der Theorie,
Schmelzpunkt: ab 150°C (Zers.)

| C₂₄H₂₈Cl₂N₆O₄S (567,50) | | | | | |
|---|---|---|---|---|---|
| Ber.: | C 50,80 | H 4,97 | N 14,81 | S 5,65 | Cl 12,49 |
| Gef.: | 50,42 | 5,47 | 14,74 | 5,38 | 12,28 |

### Beispiel 40

### 2-Carbethoxy-N-[1-(1H-benzimidazol-5-yl-methyl)-2-(4-methyl-piperidin-1-yl)-1,2,3,4-tetrahydro-isochinolin-5-sulfonamid

Hergestellt aus N-[1-(1H-Benzimidazol-5-yl-methyl)-2-(4-methyl-piperidin-1-yl)-1,2,3,4-tetrahydro-isochinolin-5-sulfon amid und Chlorameisensäure-ethylester in Methylenchlorid und Triethylamin analog Beispiel 2.
Ausbeute: 16,4 % der Theorie,
Schmelzpunkt: ab 100°C (Zers.)

| C₂₈H₃₅N₅O₅S (553,69) | | | | |
|---|---|---|---|---|
| Ber.: | C 60,74 | H 6,37 | N 12,65 | S 5,79 |
| Gef.: | 60,52 | 6,62 | 12,88 | 5,68 |

### Beispiel 41

### 2-Acetyl-N-[1-(1H-benzimidazol-5-yl-methyl)-2-(4-methyl-piperidin-1-yl)-1,2,3,4-tetrahydro-isochinolin-5-sulfonamid

Hergestellt aus N-[1-(1H-Benzimidazol-5-yl-methyl)-2-(4-methyl-piperidin-1-yl)-1,2,3,4-tetrahydro-isochinolin-5-sulfonamid und Acetanhydrid analog Beispiel 40.
Ausbeute: 29,0 % der Theorie,
Schmelzpunkt: ab 145°C (Zers.)

| C₂₇H₃₃N₅O₄S (523,66) | | | | |
|---|---|---|---|---|
| Ber.: | C 61,93 | H 6,35 N | 13,37 | S 6,12 |
| Gef.: | 61,80 | 6,60 | 13,50 | 6,01 |

### Beispiel 42

### 4-Amino-N-[1-(1H-benzimidazol-5-yl-methyl)-2-(4-methyl-1,2,3,6-tetrahydro-pyridin-1-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 4-Methyl-1,2,3,6-tetrahydro-pyridin analog Beispiel 15.
Ausbeute: 33,0 % der Theorie,
Schmelzpunkt: 117-121°C

| C₂₂H₂₃Cl₂N₅O₃S (508,43) | | | |
|---|---|---|---|
| Ber.: | C 51,97 | H 4,56 | N 13,77 |
| Gef.: | 52,19 | 4,71 | 13,41 |

### Beispiel 43

### 4-Amino-N-[1-(1H-benzimidazol-5-yl-methyl)-2-(2-carbethoxy-4-methyl-piperidin-1-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 4-Methyl-piperidin-2-carbonsäure-ethylester analog Beispiel 15.
Ausbeute: 32,0 % der Theorie,
Schmelzpunkt: ab 110°C (Zers.)

| C₂₅H₂₉Cl₂N₅O₅S (582,51) | | | | | |
|---|---|---|---|---|---|
| Ber.: | C 51,55 | H 5,02 | N 12,02 | S 5,50 | Cl 12,17 |
| Gef.: | 51,84 | 5,16 | 11,87 | 5,76 | 12,23 |

### Beispiel 44

### 4-Amino-N-[1-(1H-benzimidazol-5-yl-methyl)-2-(5,6,7,8-tetrahydro-thiazolo[4',5':5,4]thieno[3,2-c]pyridin-7-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 5,6,7,8-Tetrahydro-thiazolo[4',5':5,4]thieno[3,2-c]pyridin analog Beispiel 15.
Ausbeute: 49,0 % der Theorie,
Schmelzpunkt: 160-165°C

| C₂₄H₂₀Cl₂N₆O₃S₃ (607,56) | | | | | |
|---|---|---|---|---|---|
| Ber.: | C 47,45 | H 3,32 | N 13,83 | S 15,83 | Cl 11,67 |
| Gef.: | 47,31 | 3,66 | 13,64 | 14,13 | 11,45 |

### Beispiel 45

### 4-Amino-N-[1-(1H-benzimidazol-5-yl-methyl)-2-(4,5,6,7-tetrahydro-thieno[3,2-c]pyridin-5-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 4,5,6,7-Tetrahydro-thieno[3,2-c]pyridin analog Beispiel 15.
Ausbeute: 22,0 % der Theorie,
Schmelzpunkt: 236-238°C

| C₂₃H₂₁Cl₂N₅O₃S₂ (550,49) | | | | | |
|---|---|---|---|---|---|
| Ber.: | C 50,18 | H 3,85 | N 12,72 | S 11,65 | Cl 12,88 |
| Gef.: | 49,99 | 3,87 | 12,56 | 11,38 | 12,77 |

### Beispiel 46

### 4-Amino-N-[1-(1H-benzimidazol-5-yl-methyl)-2-(2,3,4,5,6,7-hexahydro-1H-azepin-1-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und Hexahydro-azepin analog Beispiel 15.
Ausbeute: 18,6 % der Theorie,
Schmelzpunkt: 261-265°C

| C₂₂H₂₅Cl₂N₅O₃S (510,45) | | | | | |
|---|---|---|---|---|---|
| Ber.: | C 51,77 | H 4,94 | N 13,72 | S 6,28 | Cl 13,89 |
| Gef.: | 51,77 | 5,06 | 13,85 | 6,16 | 13,70 |

### Beispiel 47

### 4-Amino-N-[1-(1H-benzimidazol-5-yl-methyl)-2-(4-phenyl-1,2,3,6-tetrahydro-pyridin-1-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 4-Phenyl-1,2,3,6-tetrahydro-pyridin analog Beispiel 15.
Ausbeute: 44,0 % der Theorie,
Schmelzpunkt: ab 175°C (Zers.)

| C₂₇H₂₅Cl₂N₅O₃S (570,50) | | | | | |
|---|---|---|---|---|---|
| Ber.: | C 56,84 | H 4,42 | N 12,28 | S 5,62 | Cl 12,43 |
| Gef.: | 56,90 | 4,50 | 12,30 | 5,81 | 12,36 |

### Beispiel 48

### 4-Amino-N-[1-(1H-benzimidazol-5-yl-methyl)-2-(4,4-dimethyl-piperidin-1-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 4,4-Dimethyl-piperidin analog Beispiel 15.
Ausbeute: 53,0 % der Theorie,
Schmelzpunkt: 247-248°C

| C₂₃H₂₇Cl₂N₅O₃S (524,47) | | | | | |
|---|---|---|---|---|---|
| Ber.: | C 52,67 | H 5,19 | N 13,35 | S 6,11 | Cl 13,52 |
| Gef.: | 52,51 | 5,26 | 13,56 | 6,30 | 13,78 |

### Beispiel 49

### 4-Amino-N-[1-(1H-benzimidazol-5-yl-methyl)-2-(2-phenyl-morpholin-4-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 2-Phenyl-morpholin analog Beispiel 15.
Ausbeute: 52,2 % der Theorie,
Schmelzpunkt: ab 230°C

| C₂₆H₂₅Cl₂N₅O₄S (574,49) | | | | | |
|---|---|---|---|---|---|
| Ber.: | C 54,36 | H 4,39 | N 12,19 | S 5,58 | Cl 12,34 |
| Gef.: | 54,14 | 4,43 | 12,07 | 5,90 | 12,56 |

### Beispiel 50

### 4-Amino-N-[1-(1H-benzimidazol-5-yl-methyl)-2-(2-amino-5,6,7,8-tetrahydro-thiazolo[4',5':5,4]thieno[3,2-c]pyridin-7-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und 2-Amino-5,6,7,8-tetrahydro-thiazolo[4',5':5,4]thieno[3,2-c]pyridin analog Beispiel 15.
Ausbeute: 58 % der Theorie,
Schmelzpunkt: 203-207°C

| C₂₄H₂₁Cl₂N₇O₃S₃ (622,58) | | | | | |
|---|---|---|---|---|---|
| Ber.: | C 46,30 | H 3,40 | N 15,75 | S 15,45 | Cl 11,39 |
| Gef.: | 46,12 | 3,71 | 15,46 | 15,67 | 11,55 |

### Beispiel 51

### N-[1-(1H-Benzimidazol-5-yl-methyl)-2-(4-methyl-piperidin-1-yl)-2-oxo-ethyl]-2'-amino-4-biphenylyl-sulfonamid

Hergestellt aus N-[1-(1H-Benzimidazol-5-yl-methyl)-2-(4-methyl-piperidin-1-yl)-2-oxo-ethyl]-2'-nitro-4-biphenylyl-sulfonamid in Gegenwart von Platin/Kohle oder Raney-Nickel und einem Wasserstoffdruck von 3 bar analog Beispiel 22.
Ausbeute: 28,0 % der Theorie,
Schmelzpunkt: ab 82°C

| C₂₈H₃₁N₅O₃S (517,66) | | | | | |
|---|---|---|---|---|---|
| Ber.: | C 55,25 | H 5,80 | N 11,51 | S 5,27 | Cl 11,65 |
| Gef.: | 55,58 | 6,22 | 11,46 | 5,52 | 11,67 |

### Beispiel 52

### 4-Amino-N-[1-(1H-benzimidazol-5-yl-methyl)-2-(2-carboxy-4-methyl-piperidin-1-yl)-2-oxo-ethyl]-3,5-dichlor-benzolsulfonamid

Hergestellt durch Hydrolyse von 4-Amino-N-[1-(1H-benzimidazol-5-yl-methyl)-2-(2-carbethoxy-4-methyl-piperidin-1-yl)-2-oxo-ethyl]-3,5-dichlor-benzolsulfonamid in Gegenwart von 1N Natronlauge analog Beispiel 24.
Ausbeute: 61,0 % der Theorie,
Schmelzpunkt: ab 180°C (Zers.)

| C₂₃H₂₅Cl₂N₅O₅S (554,46) | | | | | |
|---|---|---|---|---|---|
| Ber.: | C 49,82 | H 4,54 | N 12,63 | S 5,78 | Cl 12,79 |
| Gef.: | 49,65 | 4,56 | 12,53 | 5,41 | 13,00 |

### Beispiel 53

### N-[1-(1H-Benzimidazol-5-yl-methyl)-2-(4-methyl-piperidin-1-yl)-2-oxo-ethyl]-3-methyl-1,2,3,4-tetrahydro-chinolin-8-sulfonamid

Hergestellt durch katalytische Hydrierung von N-[1-(1H-Benzimidazol-5-yl-methyl)-2-(4-methyl-piperidin-1-yl)-2-oxo-ethyl]-3-methyl-chinolin-8-sulfonamid in Gegenwart von Palladium/Kohle in 50%iger Essigsäure und einem Wasserstoffdruck von 3 bar analog Beispiel 35.
Ausbeute: 68,0 % der Theorie,
Schmelzpunkt: ab 90°C

| C₂₆H₃₃N₅O₃S (495,65) | | | | |
|---|---|---|---|---|
| Ber.: | C 63,00 | H 6,71 | N 14,13 | S 6,47 |
| Gef.: | 62,82 | 6,74 | 14,52 | 6,20 |

### Beispiel 54

### 4-Amino-N-[1-(1H-benzimidazol-5-yl-methyl)-2-(4-carbethoxy-piperidin-1-yl)-2-oxo-ethyl]-3,5-dichlor-benzolsulfonamid

Hergestellt aus 2-[N-(4-Amino-3,5-dichlor-phenyl)-sulfamoyl]-3-(1H-benzimidazol-5-yl)-propionsäure und Piperidin-4-carbonsäure-ethylester analog Beispiel 15.
Ausbeute: 64 % der Theorie,
Schmelzpunkt: 144-148°C

| C₂₄H₂₇Cl₂N₅O₅S (568,48) | | | | | |
|---|---|---|---|---|---|
| Ber.: | C 50,71 | H 4,79 | N 12,32 | S 5,64 | Cl 12,47 |
| Gef.: | 50.55 | 4,68 | 12,32 | 5,60 | 12,71 |

### Beispiel 55

### 4-Amino-N-[1-(1H-benzimidazol-5-yl-methyl)-2-(4-carboxy-piperidin-1-yl)-2-oxo-ethyl]-3,5-dichlor-benzolsulfonamid

Hergestellt aus 4-Amino-N-[1-(1H-benzimidazol-5-yl-methyl)-2-(4-carbethoxy-piperidin-1-yl)-2-oxo-ethyl]-3,5-dichlor-benzolsulfonamid und 1N Natronlauge analog Beispiel 24.
Ausbeute: 65 % der Theorie,
Schmelzpunkt: 255-256°C (Zers.)

| C₂₂H₂₃Cl₂N₅O₅S (540,43) | | | | | |
|---|---|---|---|---|---|
| Ber.: | C 48,90 | H 4,29 | N 12,96 | S 5,93 | Cl 13,12 |
| Gef.: | 48,73 | 4,17 | 12,85 | 5,63 | 13,02 |

### Beispiel 56

### 4-Amino-N-[1-(1H-benzimidazol-5-yl-methyl)-2-(4-aminocarbonyl-piperidin-1-yl)-2-oxo-ethyl]-3,5-dichlor-benzolsulfonamid

540 mg (1 mMol) 4-Amino-N-[1-(1H-benzimidazol-5-yl-methyl)-2-(4-carboxy-piperidin-1-yl)-2-oxo-ethyl]-3,5-dichlor-benzolsulfonamid werden in 10 ml absolutem Dimethylformamid gelöst und unter Rühren mit 180 mg (1,1 mMol) Carbonyl-diimidazol versetzt. Nach etwa einer Stunde werden 2 ml äthanolische Ammoniaklösung zugetropft und 12 Stunden weiter gerührt. Anschließend wird eingedampft, der Rückstand mit Wasser versetzt und mit Essigester 2 mal extrahiert. Die organische Phase wird mit wässriger Kochsalzlösung 2 mal gewaschen, mit Natriumsulfat getrocknet und eingedampft. Der Rückstand wird auf einer Kieselgelsäule gereinigt, das eingedampfte Eluat mit Äther verrieben und abgesaugt.
Ausbeute: 170 mg (31,5 % der Theorie),
Schmelzpunkt: ab 210°C (Zers.)

| | | | | | |
|---|---|---|---|---|---|
| Ber.: | C 48,98 | H 4,48 | N 15,58 | S 5,94 | Cl 13,14 |
| Gef.: | 48,82 | 4,39 | 15,35 | 5,70 | 13,08 |

### Beispiel 57

### 4-Amino-N-[1-(1H-benzimidazol-5-yl-methyl)-2-(4-cyano-piperidin-1-yl)-2-oxo-ethyl]-3,5-dichlor-benzolsulfonamid

Hergestellt aus 4-Amino-N-[1-(1H-benzimidazol-5-yl-methyl)-2-(4-aminocarbonyl-piperidin-1-yl)-2-oxo-ethyl]-3,5-dichlor-benzolsulfonamid durch Umsetzung mit Phosphoroxychlorid bei Raumtemperatur.
Ausbeute: 70 % der Theorie,
Schmelzpunkt: 231-234°C (Zers.)

| C₂₂H₂₂Cl₂N₆O₃ (521,43) | | | | | |
|---|---|---|---|---|---|
| Ber.: | C 50,68 | H 4,25 | N 16,12 | S 6,15 | Cl 13,60 |
| Gef.: | 50,75 | 4,28 | 15,93 | 6,40 | 13,70 |

### Beispiel 58

### 4-Amino-N-[1-(1H-benzimidazol-5-yl-methyl)-2-(4-hydroxy-piperidin-1-yl)-2-oxo-ethyl]-3,5-dichlor-benzolsulfonamid

410 mg (0,8 mMol) 4-Amino-N-[1-(1H-benzimidazol-5-yl-methyl)-2-(piperidin-4-on-1-yl]-2-oxo-ethyl]-3,5-dichlor-benzolsulfon amid werden in 8 ml absolutem Dimethylformamid gelöst, mit 100 mg (2,6 mMol) Natriumborhydrid versetzt und 12 Stunden bei Raumtemperatur stehen gelassen. Anschließend wird das Reaktionsgemisch in Wasser gegossen und 3 mal mit Essigester extrahiert. Die organische Phase wird 2 mal mit Wasser gewaschen, mit Natriumsulfat getrocknet und eingedampft. Der Rückstand wird auf einer Kieselgelsäule gereinigt, das eingedampfte Eluat mit Äther verrieben und abgesaugt.
Ausbeute: 230 mg (56 % der Theorie),
Schmelzpunkt: 241-243°C (Zers.)

| C₂₁H₂₃Cl₂N₅O₄S (512,42) | | | | | |
|---|---|---|---|---|---|
| Ber.: | C 49,22 | H 4,52 | N 13,66 | S 6,26 | Cl 13,84 |
| Gef.: | 48,92 | 4,70 | 13,44 | 6,37 | 14,09 |

### Beispiel 59

### 4-Amino-N-[1-(1H-benzimidazol-5-yl-methyl)-2-(4-methyl-piperazin-1-yl)-2-oxo-ethyl]-3,5-dichlor-benzolsulfonamid

Hergestellt aus 2-(4-Amino-3,5-dichlor-benzolsulfamoyl)-2-(1H-benzimidazol-5-yl)-propionsäure und N-Methylpiperazin analog Beispiel 15.
Ausbeute: 69 % der Theorie,
Schmelzpunkt: 237-240°C (Zers.)

| | | | | | |
|---|---|---|---|---|---|
| Ber.: | C 49,32 | H 5,73 | N 16,43 | S 6,27 | Cl 13,86 |
| Gef.: | 49,31 | 5,71 | 15,37 | 6,30 | 13,55 |

### Beispiel 60

### 4-Amino-N-[1-(1H-benzimidazol-5-yl-methyl)-2-(4-methylamino-piperidin-1-yl)-2-oxo-ethyl]-3,5-dichlor-benzolsulfonamid

2,05 g 4-Amino-N-[1-(1H-benzimidazol-5-yl-methyl)-2-(piperi-din-4-on-1-yl)-2-oxo-ethyl]-3,5-dichlor-benzolsulfonamid werden in 20 ml Ethanol gelöst und mit 30 ml ethanolischer Methylaminlösung versetzt. Dann gibt man 310 mg Natriumcyanborhydrid und 240 mg Eisessig zu und rührt 4 Stunden bei Raumtemperatur Danach gibt man nochmals die halbe Menge Reduktionsmittel zu und rührt über Nacht. Der ausgefallene Niederschlag wird über eine Kieselgelsäule (Laufmittel; Methylenchlorid/Methanol/Ammoniak = 8:2:0,2) chromatographiert. Man erhält 1,2 g Produkt, das aus wenig Ethanol umkristallisiert wird.
Ausbeute: 1,05 g (46 % der Theorie),

| C₂₂H₂₆N₆Cl₂O₃S (525,46) | | | |
|---|---|---|---|
| Ber.: | C 50,44 | H 5,64 | N 14,70 |
| Gef.: | 49,54 | 5,68 | 14,62 |

## Patentansprüche

1. Benzimidazolylderivate der allgemeinen Formel 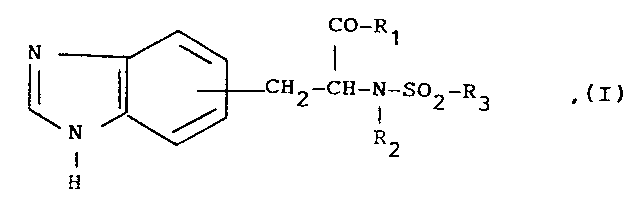 in der
R₁ eine durch Alkylgruppen disubstituierte Aminogruppe, in welcher eine Alkylgruppe durch eine Phenylgruppe substituiert sein kann,
eine gegebenenfalls durch eine Phenyl-, Hydroxy-, Carboxy-, Alkylcarbonyl-, Aminocarbonyl-, Cyano- oder N-Alkanoyl-alkylaminogruppe substituierte Pyrrolidino-, Piperidino- oder Hexamethyleniminogruppe, wobei die Hydroxygruppe nicht in α-Position zu dem Ringstickstoffatom stehen kann, eine der vorstehend erwähnten Piperidinogruppen zusätzlich durch eine Alkylgruppe substituiert und zusätzlich die Methylengruppe in 4-Stellung der Piperidinogruppe durch ein Sauerstoffatom, eine Carbonyl-, Sulfinyl-, Imino- oder N-Alkyl-iminogruppe oder eine Ethylengruppe in 3-, 4-Stellung der Piperidinogruppe oder in 4-, 5-Stellung der Hexamethyleniminogruppe durch eine Ethenylen-, Thiophenylen- oder Thiazolylengruppe ersetzt sein kann,
eine durch zwei oder drei Alkylgruppen substituierte Piperidinogruppe, in welcher die Alkylsubstituenten gleich oder verschieden sein können,
eine gegebenenfalls in 2-Stellung durch eine Aminogruppe substituierte Tetrahydro-4H-thiazolo[4,5-d]azepin-6-yl- oder Tetrahydro-thiazolo[4',5':5,4]thieno[3,2-c]pyridin-7-yl-gruppe,
R₂ ein Wasserstoffatom oder eine Alkylgruppe und
R₃ eine gegebenenfalls durch ein Fluor-, Chlor- oder Bromatom, durch eine Alkyl-, Nitro-, Amino-, Alkylamino-, Dialkylamino-, Phenyl- oder Cyclohexylgruppe substituierte Phenylgruppe, wobei der Phenylsubstituent ebenfalls durch ein Fluor-, Chlor- oder Bromatom, durch eine Nitro- oder Aminogruppe substituiert sein kann,
eine durch Fluor-, Chlor- oder Bromatome, durch Alkyl- oder Alkoxygruppen disubstituierte Phenylgruppe, wobei einer der Substituenten auch eine Nitro- oder Aminogruppe darstellen kann,
eine durch eine Hydroxy-, Amino-, Alkylamino-, Dialkylamino- oder Pyrrolylgruppe substituierte Phenylgruppe, wobei gleichzeitig die Phenylgruppe durch zwei Chlor- oder Bromatome oder durch zwei Alkylgruppen mit 1 bis 4 Kohlenstoffatomen substituiert ist und die Pyrrolylgruppe durch eine oder zwei Alkylgruppen substituiert sein kann,
eine gegebenenfalls durch Hydroxy-, Alkoxy- oder Dialkylaminogruppen mono- oder disubstituierte Naphthylgruppe,
eine gegebenenfalls durch eine Alkylgruppe substituierte Indanyl-, Chinolyl-, 1,2,3,4-Tetrahydro-chinolyl-, Isochinolyl-, 1,2,3,4-Tetrahydro-isochinolyl-, Carbazol-, 1,2,3,4-Tetrahydrocarbazol- oder Dibenzofuranylgruppe, wobei eine Iminogruppe zusätzlich durch eine Alkylgruppe, welche gleichzeitig durch eine Carboxy- oder Alkoxycarbonylgruppe substituiert sein kann, substituiert sein kann,
wobei, soweit nichts anderes erwähnt wurde, die bei der Definition der Reste R₁ bis R₃ erwähnten Alkyl-, Alkanoyl- und Alkoxyteile jeweils 1 bis 3 Kohlenstoffatome enthalten können,
deren Gemische von Stellungsisomeren und deren Salze.

2. Benzimidazolylderivate der allgemeinen Formel I gemäß Anspruch 1, in der
R₁ eine N-Methyl-benzylaminogruppe, eine gegebenenfalls in 4-Stellung durch eine Alkylgruppe mit 1 bis 3 Kohlenstoffatomen, durch eine Hydroxy-, Cyano-, Aminocarbonyl-, Methylamino- oder N-Acetyl-methylaminogruppe substituierte Piperidinogruppe, eine durch zwei oder drei Methylgruppen substituierte Piperidinogruppe, eine durch eine Carboxy-, Methoxycarbonyl- oder Ethoxycarbonylgruppe substituierte 4-Methyl-piperidinogruppe, eine gegebenenfalls in 2-Stellung durch eine Phenylgruppe substituierte Morpholinogruppe, eine 4-Oxo-pyrrolidino-, 1-Oxido-thiomorpholino-, 2,3,4,5,6,7-Hexahydro-1H-azepino-, 4-Methyl-piperazino-, 5,6,7,8-Tetrahydro-thiazolo[4',5':5,4]thieno[3,2-c]pyridino-, 4,5,6,7-Tetrahydro-thieno[3,2-c]pyridino-, 5,6,7,8-Tetrahydro-4H-thiazolo[4,5-d]azepino-, 5,6,7,8-Tetrahydro-4H-thieno[2,3-d]azepino-, 5,6,7,8-Tetrahydro-thiazolo[4',5':5,4]thieno[3,2-c]pyridino- oder 2-Amino-5,6,7,8-tetrahydro-thiazolo[4',5':5,4]thieno[3,2-c]pyr idinogruppe,
R₂ ein Wasserstoffatom oder eine Methylgruppe und
R₃ eine gegebenenfalls durch ein Chloratom, durch eine Methyl-, Nitro-, Amino-, Phenyl- oder Cyclohexylgruppe substituierte Phenylgruppe, wobei der Phenylsubstituent ebenfalls durch ein Fluoratom, durch eine Nitro- oder Aminogruppe substituiert sein kann,
eine durch Chloratome oder durch Methylgruppen disubstituierte Phenylgruppe, wobei die Substituenten gleich oder verschieden sein können und zusätzlich auch einer der Substituenten eine Nitro- oder Aminogruppe darstellen kann,
eine durch eine Hydroxy-, Amino-, Methylamino-, Ethylamino-, Dimethylamino oder Pyrrolylgruppe substituierte Phenylgruppe, wobei gleichzeitig die Phenylgruppe durch zwei Chloratome oder durch zwei Alkylgruppen mit 1 bis 4 Kohlenstoffatomen substituiert ist,
eine gegebenenfalls durch Hydroxy-, Methoxy- oder Dimethylaminogruppen mono- oder disubstituierte Naphthylgruppe,
eine gegebenenfalls durch eine Methylgruppe substituierte Indanyl-, Chinolyl-, 1,2,3,4-Tetrahydro-chinolyl-, Isochinolyl-, 1,2,3,4-Tetrahydro-isochinolyl-, Dibenzofuryl-, Carbazolyl- oder 1,2,3,4-Tetrahydro-carbazolylgruppe, wobei eine Iminogruppe zusätzlich durch eine Methyl-, Ethyl-, Methoxycarbonyl-, Ethoxycarbonyl-, Hydroxycarbonylmethyl-, Methoxycarbonylmethyl- oder Ethoxycarbonylmethylgruppe substituiert sein kann, bedeuten,
deren Enantiomere und deren Salze.

3. Benzimidazolylderivate der allgemeinen Formel I gemäß Anspruch 1, in denen der Benzimidazolylrest in 5-Stellung substituiert ist und
R₁ eine gegebenenfalls in 4-Stellung durch eine Alkylgruppe mit 1 bis 3 Kohlenstoffatomen substituierte Piperidinogruppe, eine in 2-Stellung durch eine Carboxy-, Methoxycarbonyl- oder Ethoxycarbonylgruppe substituierte 4-Methyl-piperidinogruppe, eine 4-Oxo-piperidino-, 2,3,4,5,6,7-Hexahydro-1H-azepino-, 4-Methyl-piperazino-, 4-Methyl-1,2,3,6-tetrahydro-pyridino-, 4,5,6,7-Tetrahydro-thieno[3,2-c]pyridino-, 5,6,7,8-Tetrahydro-4H-thiazolo[4,5-d]azepino- oder 5,6,7,8-Tetrahydro-4H-thieno[2,3-d]azepino-gruppe,
R₂ ein Wasserstoffatom und
R₃ eine durch eine Hydroxy-, Amino-, Methylamino-, Ethylamino-, Dimethylamino- oder Pyrrolylgruppe substituierte Phenylgruppe, wobei gleichzeitig die Phenylgruppe durch zwei Chloratome oder durch zwei tert.Butylgruppen substituiert ist,
eine 4-Biphenylylgruppe,
eine durch eine Dimethylaminogruppe substituierte Naphthylgruppe,
eine gegebenenfalls durch eine Methylgruppe substituierte 1,2,3,4-Tetrahydro-chinolyl-, Carbazolyl-, 1,2,3,4-Tetrahydrocarbazol- oder Dibenzofurylgruppe, wobei eine Iminogruppe zusätzlich durch eine Methyl-, Methoxycarbonyl-, Ethoxycarbonyl-, Hydroxycarbonylmethyl-, Methoxycarbonylmethyl- oder Ethoxycarbonylmethylgruppe substituiert sein kann, bedeuten,
deren 1-, 3-Isomerengemische, deren Enantiomere und deren Salze.

4. Folgende Verbindungen der allgemeinen Formel I gemäß Anspruch 3:
(a) 4-Amino-N-[1-(1H-benzimidazol-5-yl-methyl)-2-(4-ethyl-piperidin-1-yl)-2-oxo-ethyl]-3,5-dichlor-benzolsulfonamid,
(b) N-[1-(1H-Benzimidazol-5-yl-methyl)-2-(4-ethyl-piperidin-1-yl)-2-oxo-ethyl]-3,5-dichlor-4-ethylamino-benzolsulfonamid,
(c) 4-Amino-N-[1-(1H-benzimidazol-5-yl-methyl)-2-(4-methyl-piperazin-1-yl)-2-oxo-ethyl]-3,5-dichlor-benzolsulfonamid,
(d) N-[1-(1H-Benzimidazol-5-yl-methyl)-2-(4-methyl-piperidin-1-yl)-2-oxo-ethyl]-9-methyl-1,2,3,4-tetrahydro-carbazol-6-sulfonamid,
(e) 4-Amino-N-[1-(1H-benzimidazol-5-yl-methyl)-2-(4,5,6,7-tetrahydro-thieno[3,2-c]pyridin-5-yl)-2-oxo-ethyl]-3,5-dichlor- benzolsulfonamid,
(f) 4-Amino-N-[1-(1H-benzimidazol-5-yl-methyl)-2-(piperidin-4-on-1-yl)-2-oxo-ethyl]-3,5-dichlor-benzolsulfonamid,
(g) N-[1-(1H-Benzimidazol-5-yl-methyl)-2-(4-methyl-piperidin-1-yl)-2-oxo-ethyl]-1,2,3,4-tetrahydro-chinolin-8-sulfonamid,
(h) 4-Amino-N-[1-(1H-benzimidazol-5-yl-methyl)-2-(2,3,4,5,6,-7-hexahydro-1H-azepin-1-yl)-2-oxo-ethyl]-3,5-dichlor-benzolsulfonamid,
(i) N-[1-(1H-Benzimidazol-5-yl-methyl)-2-(4-methyl-piperidin-1-yl)-2-oxo-ethyl]-9-ethyl-carbazol-3-sulfonamid und
(j) 4-Amino-N-[1-(1H-benzimidazol-5-yl-methyl)-2-(4-methyl-piperidin-1-yl)-2-oxo-ethyl]-3,5-dichlor-benzolsulfonamid,
deren 1-, 3-Isomerengemische, deren Enantiomere und deren Salze.

5. Physiologisch verträgliche Salze der Verbindungen nach mindestens einem der Ansprüche 1 bis 4 mit anorganischen oder organischen Säuren oder Basen.

6. Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 5 zur Herstellung eines Arzneimittels mit einer die Thrombinzeit verlängernden Wirkung, einer thrombinhemmenden Wirkung und einer Hemmwirkung auf verwandte Serinproteasen.

8. Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

9. Verfahren zur Herstellung der Benzimidazole gemäß den Ansprüchen 1 bis 5, dadurch gekennzeichnet, daß
a) eine Verbindung der allgemeinen Formel 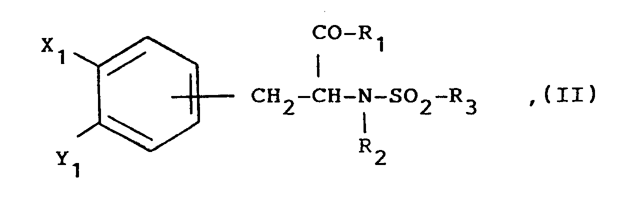 in der
R₁, R₂ und R₃ wie in den Ansprüchen 1 bis 5 definiert sind,
einer der Reste X₁ oder Y₁ eine Formylaminogruppe und
der andere der Reste X₁ oder Y₁ eine Aminogruppe darstellen, cyclisiert wird oder
b) eine Verbindung der allgemeinen Formel 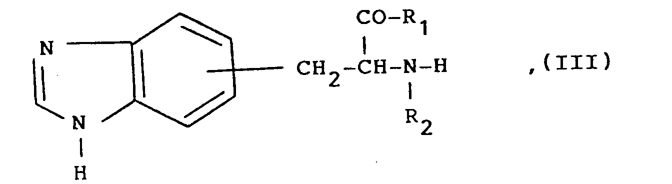 in der
R₁ und R₂ wie in den Ansprüchen 1 bis 5 definiert sind, mit einer Verbindung der allgemeinen Formel
Z₁-SO₂-R₃ , (IV)
in der
R₃ wie in den Ansprüchen 1 bis 5 definiert ist und
Z₁ eine nukleophile Austrittsgruppe darstellt, umgesetzt wird oder
c) eine Verbindung der allgemeinen Formel 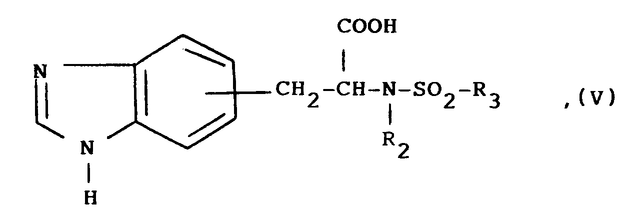 in der
R₂ und R₃ wie in den Ansprüchen 1 bis 5 definiert sind, oder dessen reaktionsfähiges Derivat mit einer Verbindung der allgemeinen Formel
H-R₁ , (VI)
in der
R₁ wie in den Ansprüchen 1 bis 5 definiert ist, umgesetzt wird oder
d) zur Herstellung von Verbindungen der allgemeinen Formel I, in der R₃ eine Alkylamino- oder Dialkylaminogruppe enthält, eine Verbindung der allgemeinen Formel 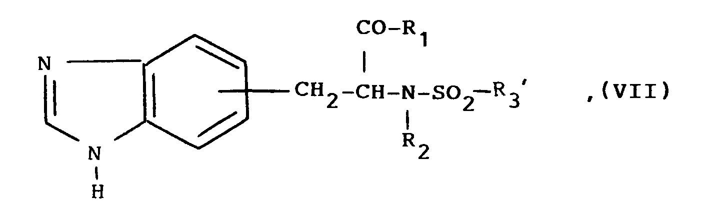 in der
R₁ und R₂ wie in den Ansprüchen 1 bis 5 definiert sind und
R₃' eine durch eine Amino- oder Alkylaminogruppe substituierte Phenylgruppe, die zusätzlich durch zwei Chlor- oder Bromatome oder durch zwei Alkylgruppen mit 1 bis 4 Kohlenstoffatomen substituiert sein kann, darstellt, mit einem Alkanal mit 1 bis 3 Kohlenstoffatomen reduktiv aminiert wird
und gewünschtenfalls anschließend eine so erhaltene Verbindung der allgemeinen Formel I, in der R₃ eine Aminogruppe enthält, mittels Umsetzung eines entsprechenden Furans in eine entsprechende Verbindung der allgemeinen Formel I, in der R₃ einen entsprechenden Pyrrolylrest enthält, übergeführt wird oder
eine so erhaltene Verbindung der allgemeinen Formel I, in der R₃ eine Nitrogruppe enthält, mittels Reduktion in eine entsprechende Verbindung der allgemeinen Formel I, in der R₃ eine Aminogruppe enthält, übergeführt wird oder
eine so erhaltene Verbindung der allgemeinen Formel I, die eine veresterte Carboxygruppe enthält, mittels Hydrolyse in eine entsprechende Carboxyverbindung übergeführt wird oder
eine so erhaltene Verbindung der allgemeinen Formel I, in der R₁ eine durch eine Aminocarbonylgruppe substituierte Pyrrolidino-, Piperidino- oder Hexamethyleniminogruppe darstellt, mittels Dehydratisierung in eine entsprechende Cyanoverbindung übergeführt wird oder
eine so erhaltene Verbindung der allgemeinen Formel I, in der R₁ oder R₂ oder R₁ und R₂ eine Carbonylgruppe enthält, mittels Reduktion in eine entsprechende Hydroxymethylenverbindung übergeführt wird oder
eine so erhaltene Verbindung der allgemeinen Formel I, in der R₃ einen ankondensierten Pyridinring enthält, mittels katalytischer Hydrierung in eine entsprechende Tetrahydro-Verbindung übergeführt wird oder
ein so erhaltenes 1-, 3-Isomerengemisch einer Verbindung der allgemeinen Formel I mittels Isomerentrennung in ihr 1- und 3-Isomer aufgetrennt wird oder
ein so erhaltenes Racemat einer Verbindung der allgemeinen Formel I in ihre Enantiomeren aufgetrennt wird oder
eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.
